# EUROPEAN PATENT SPECIFICATION

(11) **EP 4 463 570 B1**
(45) Date of publication and mention of the grant of the patent: **14.01.2026**
(21) Application number: 23706601.4
(22) Date of filing: 21.02.2023
(51) Int. Cl.: C12Q 1/6886, C12N 5/00, C12N 5/09

(54) **METHOD FOR HORMONE OR DRUG SCREENING IN A TISSUE SAMPLE**
VERFAHREN ZUM HORMON- ODER WIRKSTOFF-SCREENING IN EINER GEWEBEPROBE
PROCÉDÉ DE CRIBLAGE D'HORMONES OU DE MÉDICAMENTS DANS UN ÉCHANTILLON DE TISSU

(30) Priority: 22.02.2022 EP 22158098
(43) Date of publication of application: 20.11.2024
(73) Proprietor: ECOLE POLYTECHNIQUE FEDERALE DE LAUSANNE (EPFL), 1015 Lausanne (CH)
(72) Inventor: ZHANG, Yueyun, 1007 LAUSANNE (CH); MERTEN, Christoph, 1028 PREVERENGES (CH); BRISKEN, Cathrin, 1066 EPALINGES (CH)
(74) Representative: GatesIP
(86) International application number: PCT/EP2023/054320
(87) International publication number: WO 2023/161231

(56) References cited:
- WO-A1-2021/159162
- CARTAXO ANA LUÍSA ET AL: "A novel culture method that sustains ER[alpha] signaling in human breast cancer tissue microstructures", vol. 39, no. 1, 17 August 2020 (2020-08-17), XP055947254, Retrieved from the Internet <URL:https://link.springer.com/content/pdf/10.1186/s13046-020-01653-4.pdf> DOI: 10.1186/s13046-020-01653-4
- GODUGU CHANDRAIAH ET AL: "AlgiMatrix(TM) Based 3D Cell Culture System as an In-Vitro Tumor Model for Anticancer Studies", PLOS ONE, vol. 8, no. 1, 18 January 2013 (2013-01-18), pages e53708, XP055947375, DOI: 10.1371/journal.pone.0053708
- WANG C ET AL: "RNA extraction from polysaccharide-based cell-laden hydrogel scaffolds", ANALYTICAL BIOCHEMISTRY, ACADEMIC PRESS, AMSTERDAM, NL, vol. 380, no. 2, 15 September 2008 (2008-09-15), pages 333 - 334, XP023183025, ISSN: 0003-2697, [retrieved on 20080610], DOI: 10.1016/J.AB.2008.06.005
- KÖSTER NATASCHA ET AL: "Single-Step RNA Extraction from Different Hydrogel-Embedded Mesenchymal Stem Cells for Quantitative Reverse Transcription-Polymerase Chain Reaction Analysis", vol. 22, no. 6, 1 June 2016 (2016-06-01), US, pages 552 - 560, XP055948070, ISSN: 1937-3384, Retrieved from the Internet <URL:https://www.liebertpub.com/doi/pdfplus/10.1089/ten.TEC.2015.0362> DOI: 10.1089/ten.tec.2015.0362

## Description

### FIELD OF THE INVENTION

The invention relates to a hormone and drug screening method. The present invention relates to a method for analyzing cellular responsiveness to hormones or drugs in a tissue sample based on an alginate-based 3D ex vivo culture system for normal and malignant tissue microstructures and uses thereof.

### BACKGROUND OF THE INVENTION

Current drug screening platform are mostly based on 2D cell lines or 3D spheroids or organoids that are derived from single cells. However, 2D cell lines culture does not mimic the natural structures of tissues and physiological environment in vivo. 3D spheroid or organoid requires cell culture from single cells to a 3D-structured state, which involves selection of some specific cell colonies that lead to loss of tumor heterogeneity and architecture, also the animal-derived culture matrix Matrigel^{®} has high batch-to-batch variation and faces global shortage. Also, this approach fails to maintain hormone receptor expression and hormone response which is a really important aspect to take into account in some therapies.

For instance, more than 70% of breast cancers are Estrogen Receptor positive (ER+) and treated with endocrine therapy. The current standard endocrine therapies overlook that different patients have different hormone response status and drug response profiles that may affect therapeutic outcomes.

In order to maintain hormone and drug response, a solution would be to use ex vivo assays using tissue samples from a patient. However, for preclinical screening applications and personalizing cancer therapy, such as breast cancer therapy, this approach is not viable because of the large size of tissue samples that are required to feed the ex vivo assays.

Apart from the difficulty in ex vivo tissue culture with physiologically relevant hormone status, high throughput transcriptomics-based screening system for 3D tumor ex vivo, for instance 3D ER+ breast tumor ex vivo is still lacking and challenging. Three-dimensional tissue structures present more complex components than single cells and are more difficult in cell lysis and mRNA capture especially when biomaterials are incorporated in the system that can potentially block the access of cells to reagents.

In Tanos, Tamara, et al.¹, the authors studied progesterone (PR) signaling of normal breast tissue ex vivo in the form of microstructures (instead of single cells) to preserve intact tissue architectures and to enable cell-cell contact required in paracrine signaling. However, the authors did not provide structural support for the tissue microstructures and the viability of tissue dropped on day 9.

In Cartaxo, Ana Luisa, et al.², the authors developed 3D alginate-based ex vivo culture of human breast cancer microstructures for 1 month. However, the authors added supplementary hormones and growth factors, which can induce artificial hormone stimulation and disrupt the results. Also, the authors cultured only breast cancer tissue, which is less challenging than normal tissue culture because the immortal cancer cells survive better in ex vivo culture. Moreover, the authors involved a dynamic culture system with agitation, which can apply too much shear stress to the tissue and does not represent physiologically relevant environment for tissue in vivo. The authors cultured the breast tissue in alginate for 1 month, while the present invention aim to provide a fast screening that requires short term culture for usually 24h and no longer than 2 weeks.

In this method, the alginate structure is dissociated together with tissue lysis.

Chandraiah G. et al. and WO2012159162 A1 describe an alginate dissociation step before cell lysis.

In Ethan S. Sokol et al³, the authors engineered a hydrogel scaffold that incorporated both the protein (collagen, laminins, and fibronectin) and carbohydrate components (hyaluronan) of human breast tissue to enable tissue self-organization, expansion, and differentiation to form mature mammary. However, the authors used a collagen-based ECM like hydrogel, which involves many complex components of biomaterial (e.g. collagen, laminin, etc) while the present invention aim at providing a reproducible, cleaner, simpler and universal-to-use culture system. Also, the authors added supplementary hormones and growth factors (insulin, hydrocortisone, EGF), which can induce artificial hormone stimulation and disrupt the results.

Wang C. et al. and Köster N. et al. describe poor RNA extraction from hydrogel-embedded cells or tissues (including alginate), which negatively affects RT-PCR efficiency.

As such, there is a need for a method that can provide faithful recapitulation of original patient tissues and their hormone and drug response profiles to enable more accurate prediction of efficient therapeutic options.

### SUMMARY OF THE INVENTION

To overcome the challenges above, we provide herein a novel method for analyzing cellular responsiveness to hormones or drugs in a tissue sample. Said method comprises a miniaturized alginate-based 3D ex vivo culture system integrating tissue lysis, mRNA capture and reverse transcription in one step.

After mechanical and enzymatic digestion of a tissue sample from a patient, the usual step of dissociating tissue into single cells is omitted in order to obtain a "tissue microstructure", where cell-cell contact and cell surrounding environment or niche are maintained to mimic the physiological environment. Tissue microstructures are then mixed with natural biomaterial alginate and crosslinked into spheres to provide structural support and mechanical environment for the tissue. Surprisingly, said alginate-based 3D ex vivo culture system for normal and malignant tissue microstructures, such as breast tissue, can maintain the most important features of the tissue, hormone receptors and hormone responses, in basic culture medium. Said alginate-based 3D ex vivo culture system can also avoid induction of artificial hormone traces from external hormone and growth factor supplement and thus simplify the process and lower the culture cost.

The inventors were able to enable this miniaturized approach by integrating tissue lysis, mRNA capture and reverse transcription in one step. To date, this "one-step" approach which minimizes loss of material has only been done in single cell systems. The reason being that complex system with tissue microstructures and alginate present way more challenges, because they comprise more complex components such as collagens and large cell debris that can inhibit the process. Also, the alginate is a potential barrier for cells to be exposed to reagents and thus can decrease the process efficiency. As such, the problems of using said one step approach in a complex culture system have never been overcome in the prior art.

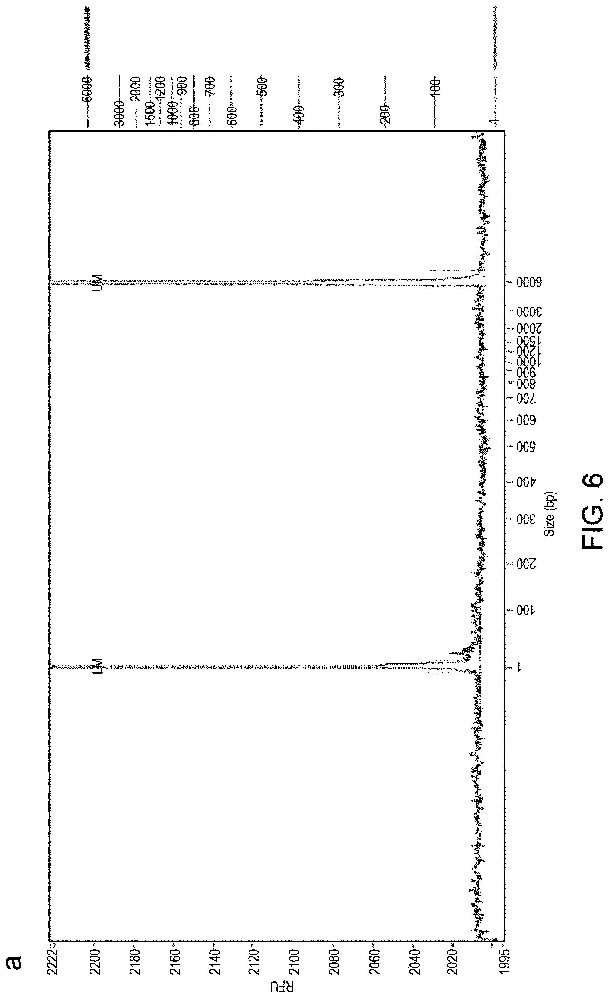

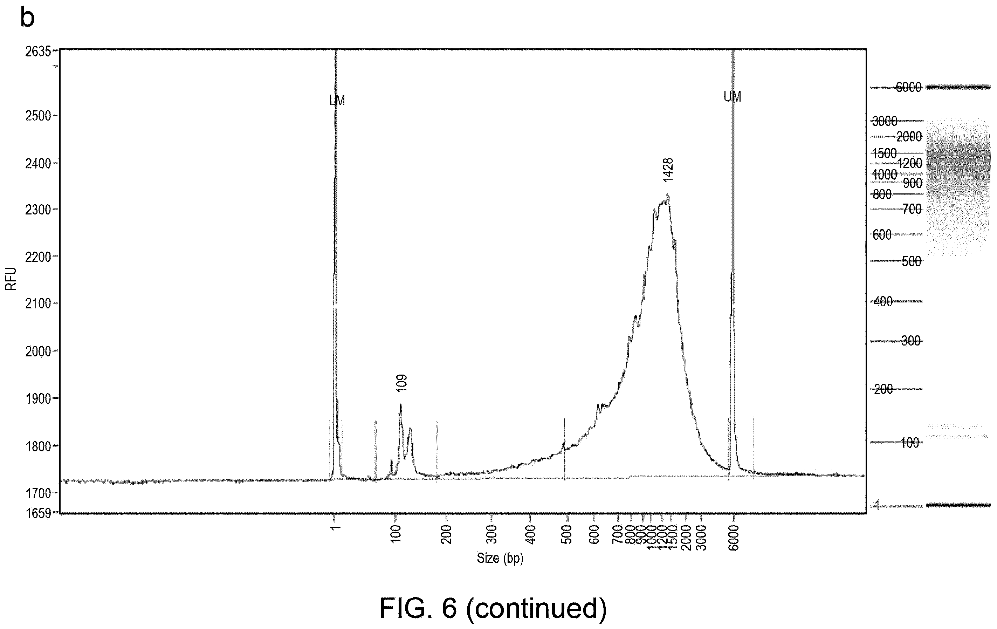

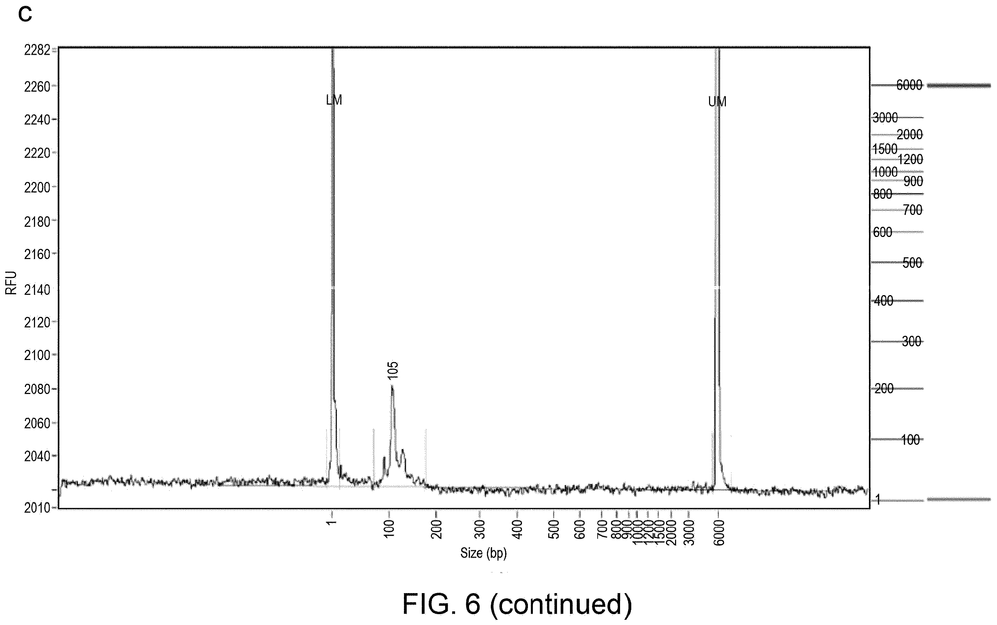

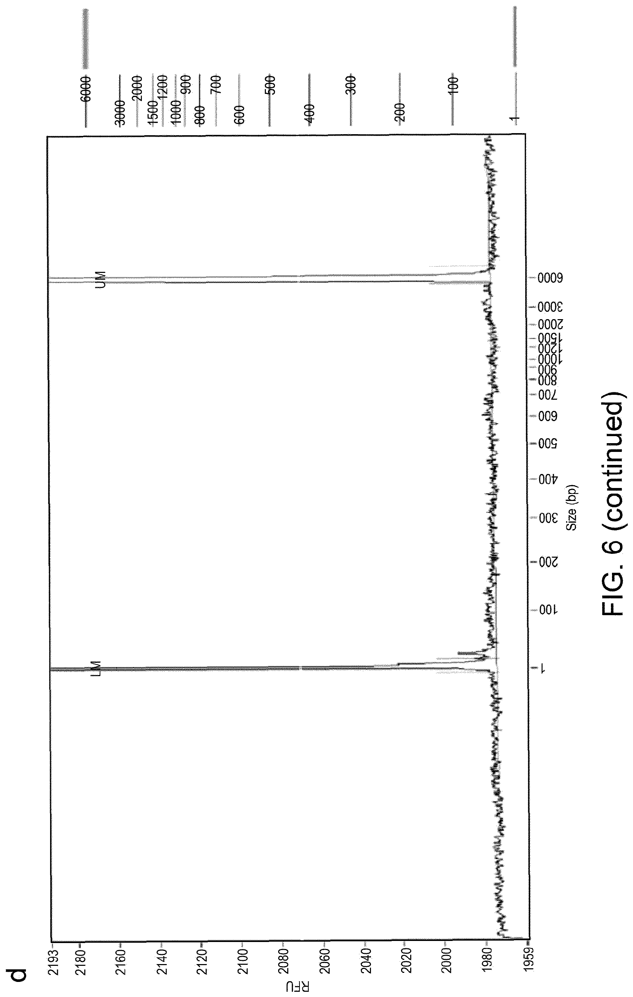

The present inventors surprisingly overcame this hurdle by bypassing the normal alginate dissociation step which is routine in research and literature with alginate. Instead, the present inventors lysed the cells and performed mRNA capture and cDNA synthesis when alginate was intact, and surprisingly, cDNA was harvested with good quantity and quality. In comparison, it was not possible to retrieve mRNA and synthesize cDNA when using conventional way of alginate dissociation before cell lysis (see Example 6 and Fig. 6).

Having enabled the one-step mRNA capture and reverse transcription in a complex system with tissue microstructures and alginate, it was then also possible to incorporate DNA barcoding strategy into the system and realize transcriptomics-based readout with RNAseq for more reliable prediction of drug effectiveness according to the up-or down-regulation of target genes.

As such, the method of the present disclosure overcomes the challenge of ex vivo tissue culture, such as ex vivo ER+ breast tissue culture and enables miniatured transcriptomics-based compound screening with high throughput and reliable prediction of drug effects. In face of global shortage of animal-derived Matrigel^{®} for PDO culture, the system also offers an animal-free, reproducible, and affordable solution for tissue culture ex vivo.

### DETAILED DESCRIPTION

### Brief description of the Figures

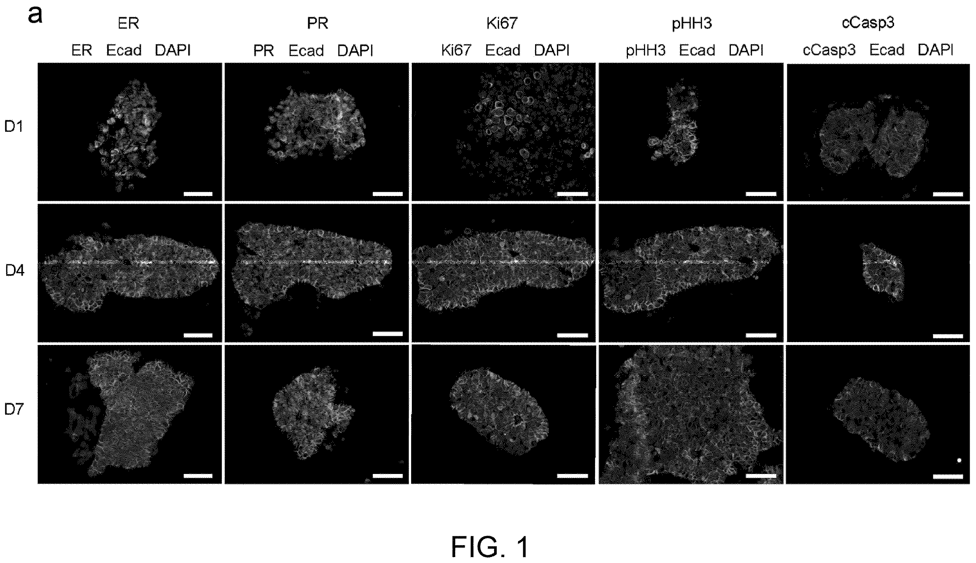

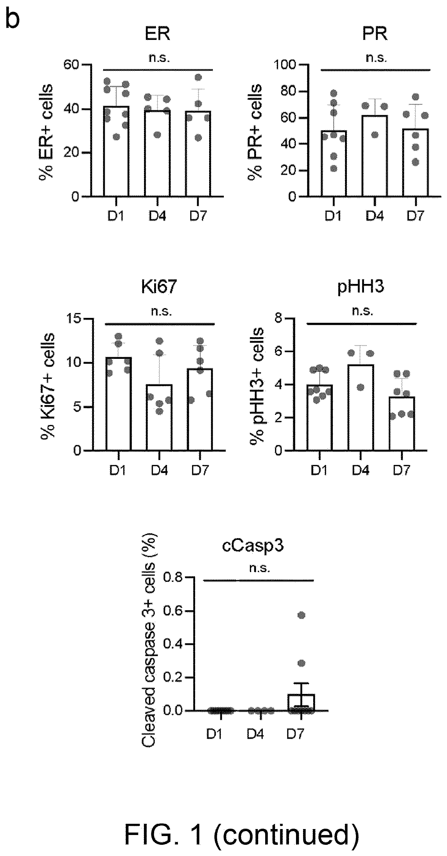

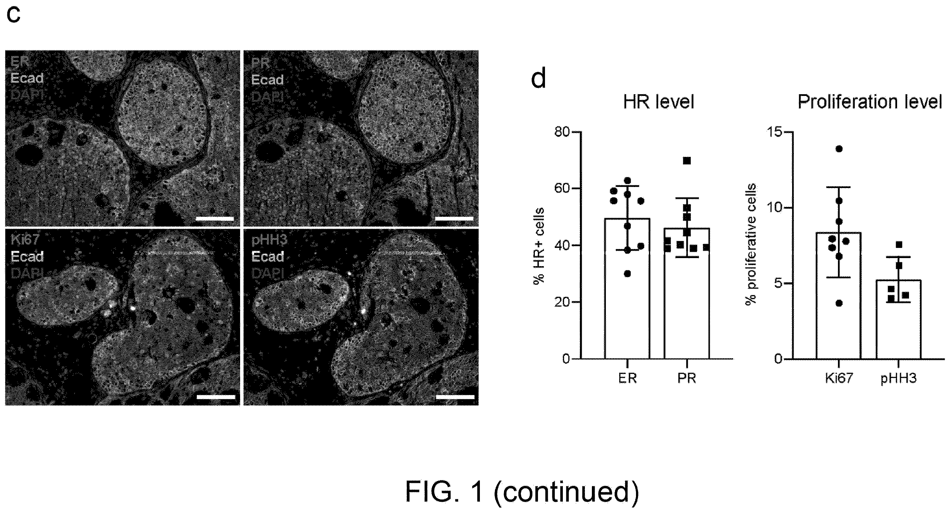

**Figure 1****. Alginate encapsulated breast tumor microstructures preserves hormone receptor expression and maintain cell proliferation for 7 days ex vivo.**
   **(a)** Fluorescent Immunohistochemistry staining of alginate encapsulated patient derived tumor microstructures on 4 µm paraffin-embedded sections with hormone receptors (ER and PR), proliferation markers (Ki67 and pHH3) and apoptosis marker (Cleaved caspase 3) in red. Sections were labeled with human specific E-cadherin (green) to identify patient cells and stained with DAPI (blue) to detect cell nuclei. Scale bars: 50 µm.
   **b)** Quantification of cells positive for human specific proliferation and apoptosis markers and hormone receptors over total numbers of cells stained with DAPI. Data represent mean ± SD, Krustal-Wallis test (non-parametric), ns. not significant.
   **c)** Fluorescent IHC of PDX gland sections labeled with human specific E-cadherin (green) to identify patient cells and stained with DAPI (blue) to detect cell nuclei. Scale bars: 100 µm.
   **d)** Quantification of cells positive for human specific ER and PR over total numbers of cells stained with DAPI to indicate hormone receptors and proliferation levels of PDX of origin. Data represent mean ± SD, unpaired Student t-test, two-tailed. *p < 0.05.

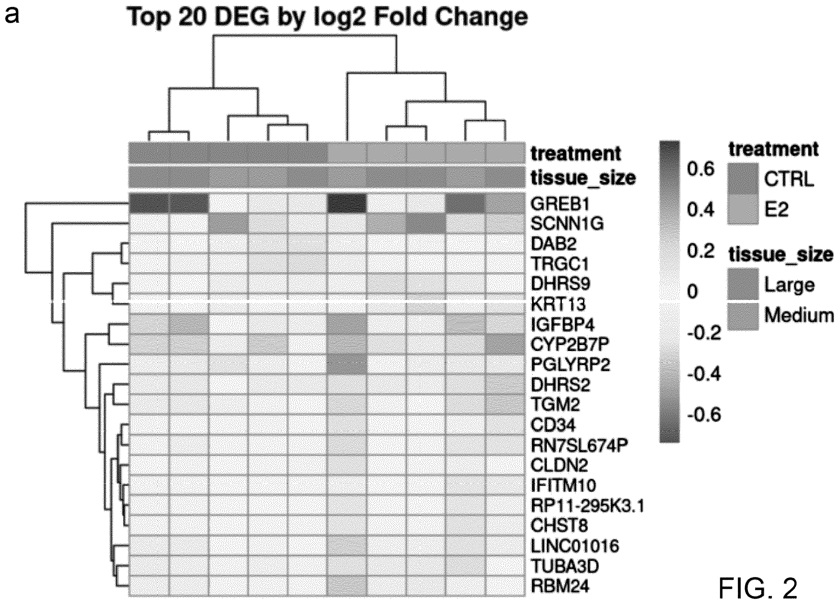

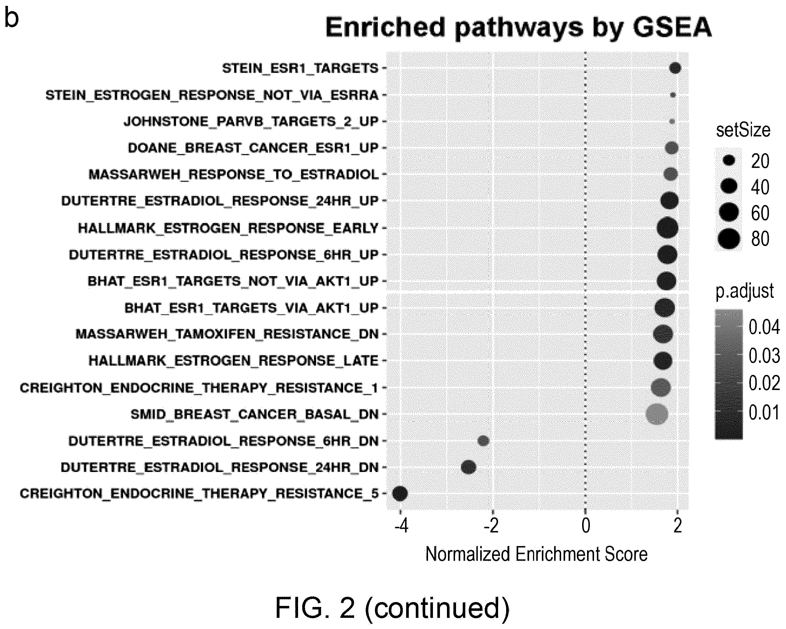

**Figure 2****. Tumor microstructures in alginate respond to both hormone receptor agonist E2 at transcriptional level, demonstrating the functionality of hormone receptors ex vivo.**
   **a)** Heat map showing top 20 differentially expressed genes sorted by log2(Fold change) for tumor microstructures with large and medium sizes encapsulated in alginate, before and after 10 nM E2 treatment for 24 hours.
   **b)** Gene sets enriched in tumor microstructures, both large and medium sizes, after 10 nM E2 treatment in alginate.

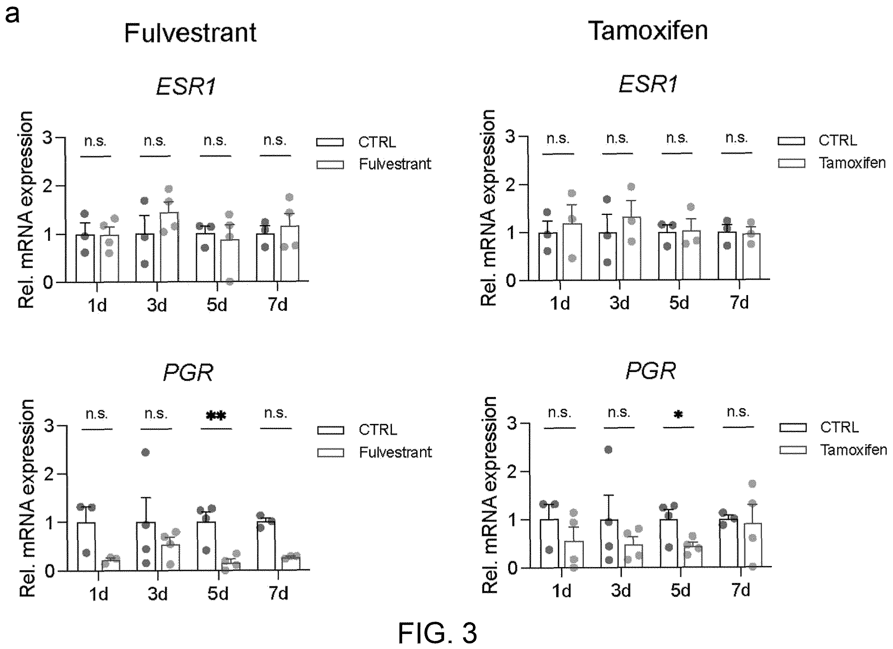

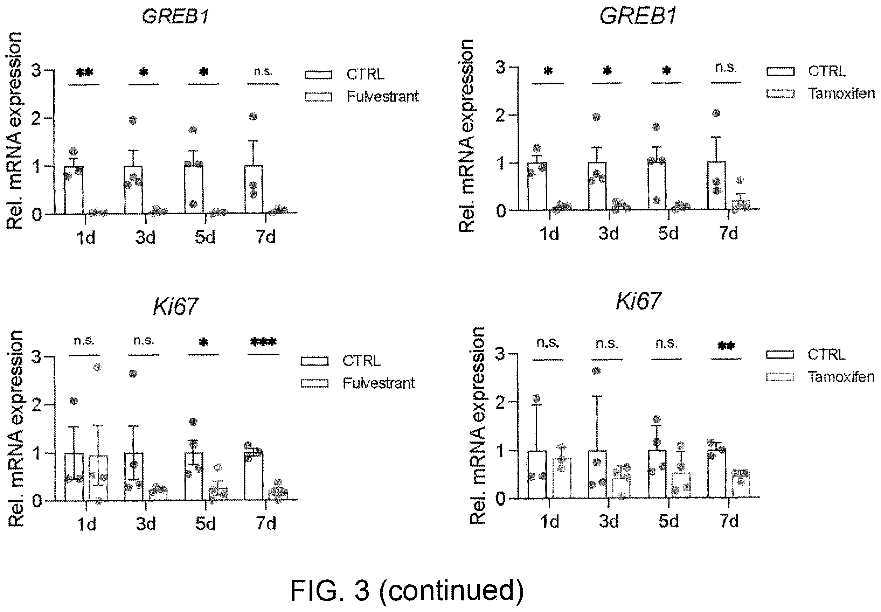

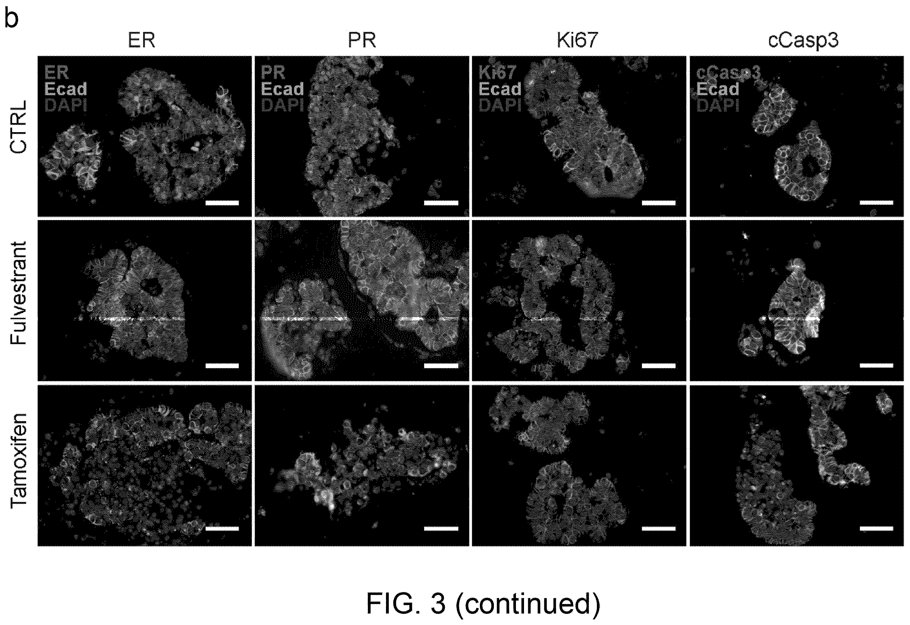

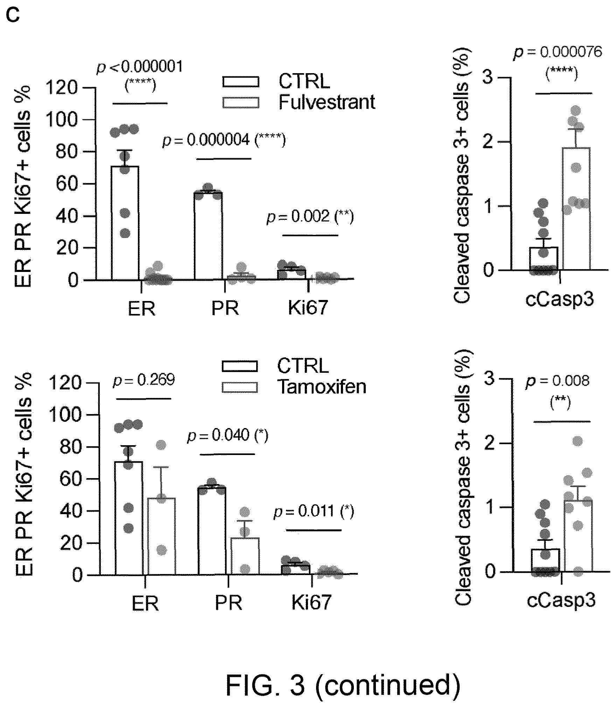

**Figure 3****. Tumor microstructures in alginate respond to selective ER degrader (fulvestrant) and selective ER modulator (tamoxifen) during 7 days at both transcriptional and protein levels.**
   **a)** Bar plots showing relative transcriptional levels of ER target genes, ESR1, PGR, GREB1, and proliferation marker Ki67 upon 100 nM fulvestrant and 200 nM tamoxifen treatment for tumor microstructures in alginate during 7 days. Data represent mean ± SEM. Multiple unpaired t-test. *p < 0.05, **p < 0.01, n.s. not significant.
   **b)** Representative immunofluorescent images for human-specific E-cadherin (green) and ER, PR, Ki67 and cleaved caspase 3 (red) counterstained with DAPI (blue) of METS15 microstructures in alginate treated with 100 nM fulvestrant or 200 nM tamoxifen for 3 days. Scale bars, 50 µm.
   **c)** Bar plots showing percentage of ER+, PR+, Ki67+ and cleaved caspase 3+ cells over total numbers of human-specific cells from microstructures in alginate treated with 100 nM fulvestrant or 200 nM tamoxifen for 3 days. Data represent mean ± SEM. Multiple unpaired t-test. *p < 0.05, **p < 0.01, ****p < 0.0001, n.s. not significant.

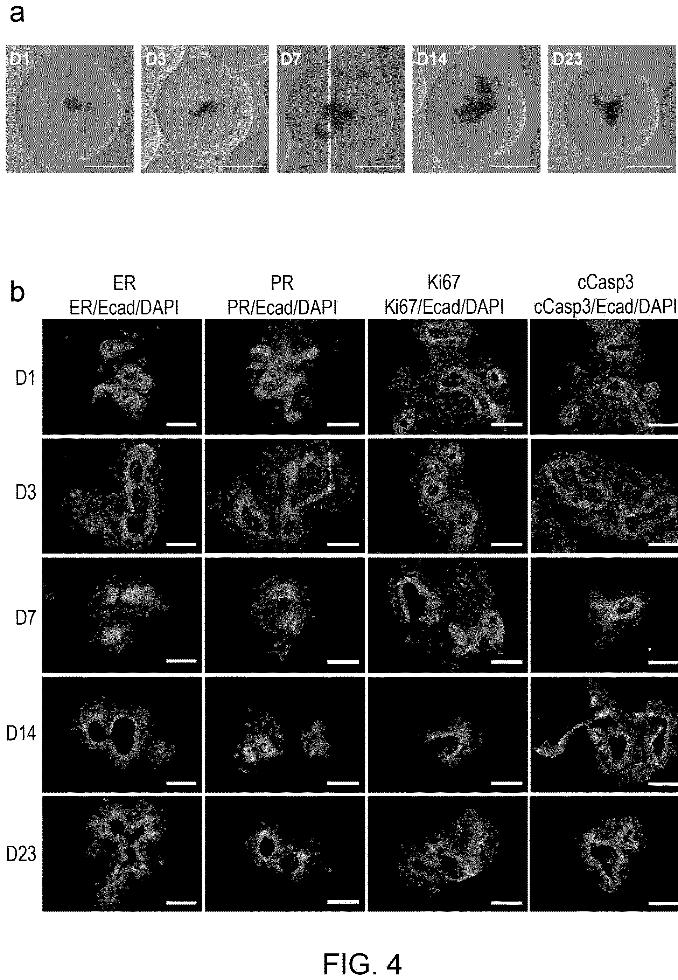

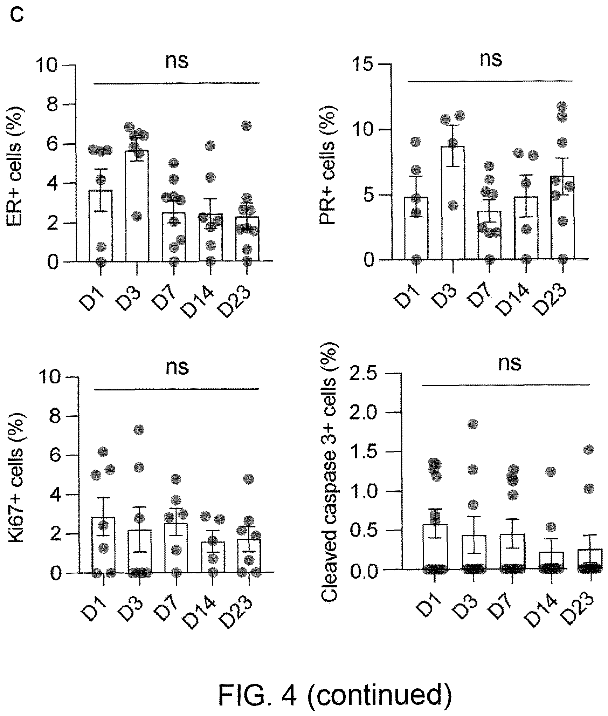

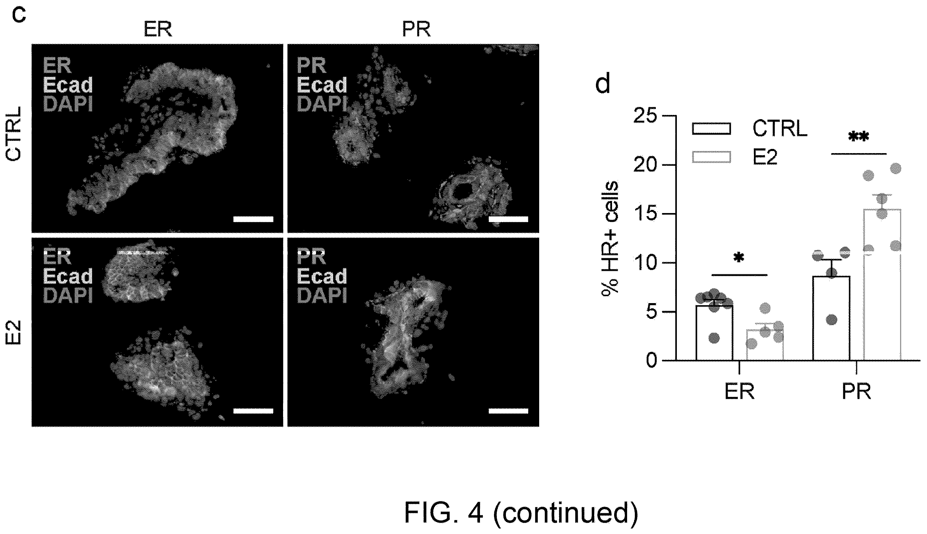

**Figure 4****. Alginate encapsulated primary normal breast tissue microstructures preserves hormone receptor expression and maintain cell proliferation for 23 days ex vivo.**
   **(a)** Representative images of primary normal breast tissue microstructures in alginate cultured for 1, 3-, 7-, 14- and 23-days ex vivo. Scale bar: 500 µm.
   **(b)** Immunofluorescent staining of alginate encapsulated primary normal breast tissue microstructures on 4 µm paraffin-embedded sections with hormone receptors (ER and PR), proliferation marker (Ki67) and apoptosis marker (Cleaved caspase 3). Sections were labeled with human specific E-cadherin and stained with DAPI to detect cell nuclei. Scale bars: 50 µm.
   **(c)** Percentage of cells positive for hormone receptors, proliferation and apoptosis markers over total numbers of human cells stained with DAPI. No significant decrease was detected in hormone receptor expression and proliferation of normal tissue microstructures in alginate over 23 days culture ex vivo. Data represent mean ± SEM, one-way ANOVA, n.s. not significant.
   **(d)** Representative immunofluorescent images for ER and PR counterstained with DAPI of primary normal breast tissue microstructures in alginate treated with 10 nM E2 for 3 days. Primary normal breast tissue responded to E2 stimulation with down-regulation of ER protein and up-regulation of PR protein expressions. Scale bars: 50 µm.
   **(e)** Quantification of cells positive for ER and PR over total numbers of cells stained with DAPI to indicate hormone receptors and proliferation levels. Data represent mean ± SEM, multiple unpaired t-test. *p < 0.05.

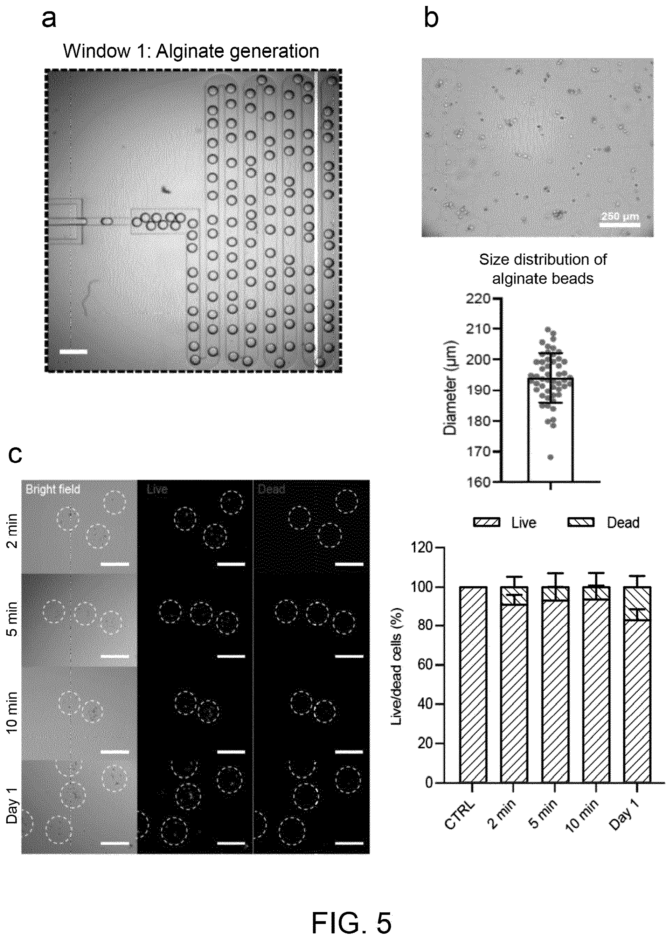

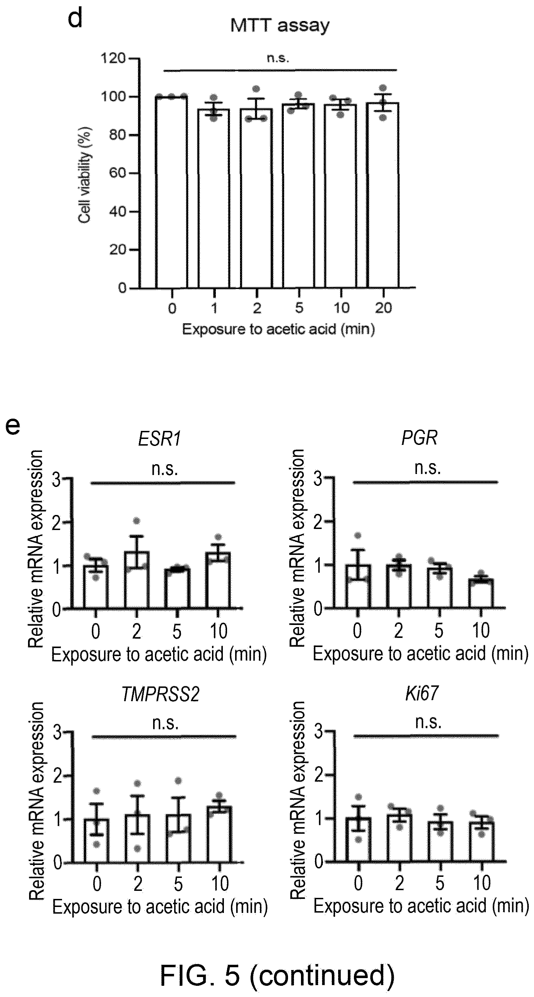

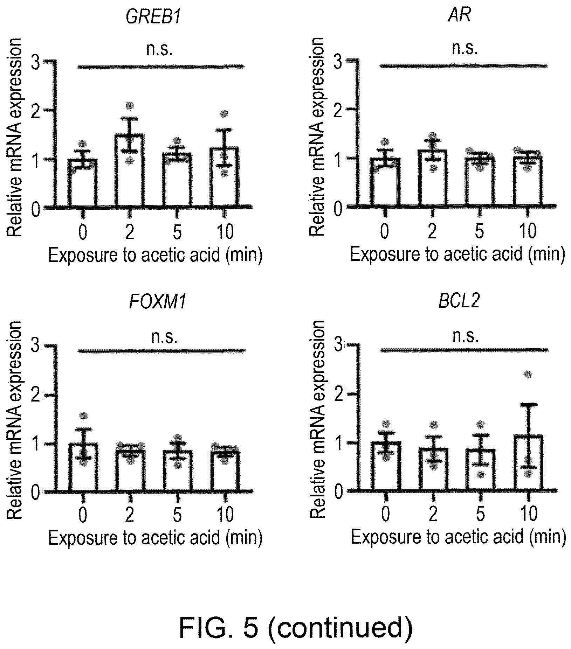

**Figure 5****. Alginate beads containing cells can be generated on microfluidic chip with high size and shape uniformity and high cell viability.**
   **a)** Micrograph and size distribution of alginate beads, with average diameter of 195 µm and high size uniformity (Scale bar, 250 µm. Data represent mean ± SD) of a microfluidic preparation (not shown) of cell-containing alginate beads generated at the flow focusing geometry and crosslinked in the delay channel with released calcium ions from EDTA-Ca and collected at the outlet.
   **b)** Cell viability determined by live/dead (Calcein-AM/Ethidium homodimer) staining at 2, 5, 10 min post-collection and after being cultured for 1 day. Scale bars, 300 µm. Data represent mean ± SD.
   **c)** Cytotoxicity of acetic acid to 2D monolayer culture of MCF7 cells with 1, 2, 5, 10, 20 min exposure determined by MTT assay. Data represent mean ± SEM.
   **d)** Relative transcript levels of hormone receptor target genes (ESR1, PGR, GREB1, AR, TMPRS S2), proliferation (Ki67) and apoptosis markers (BCL2) after 2, 5, 10 min exposure to acetic acid and cell culture for 24 h determined by qPCR. Data represent mean ± SEM.
**Figure 6****. Different strategies of cell lysis, mRNA capture and reverse transcription for cDNA synthesis of breast tissue in alginate.**
   **a)** Dissociation of alginate before one-step cell lysis, mRNA capture and reverse transcription resulted in no yield of cDNA. Dissociation of alginate before cell lysis is a standard in literatures with conventional way of cell lysis and mRNA extraction with e.g. Trizol and spin column. The one-step cell lysis, mRNA capture and RT reaction was shown to work in single cells but not in complex cell microstructures in biomaterial alginate.
   **b)** According to the present invention, no dissociation of alginate before one-step cell lysis, mRNA capture and reverse transcription, combined with dissociation of alginate after RT process, surprisingly resulted in cDNA with high yield and desired size at around 1400 bp.
   **c)** No dissociation of alginate before one-step cell lysis, mRNA capture and reverse transcription, no dissociation of alginate after RT process, resulted in no cDNA and residual of primers at smaller size around 100 bp.
   **d)** The separation of the one-step process into two steps: 1. cell lysis and mRNA capture when alginate is intact, and 2. taking the supernatant surrounding the alginate beads for the RT reaction, resulted in no cDNA.

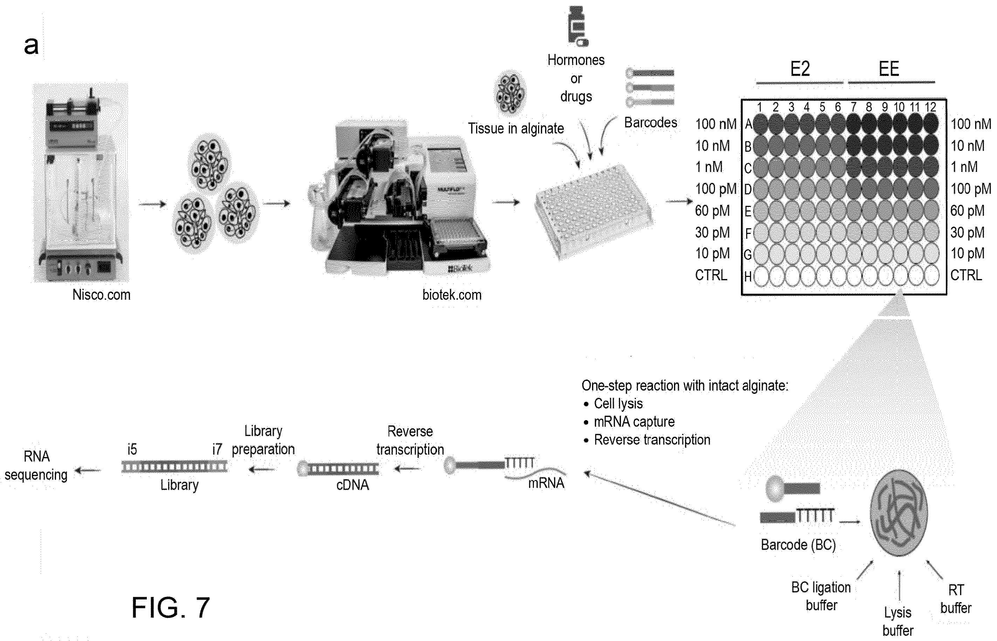

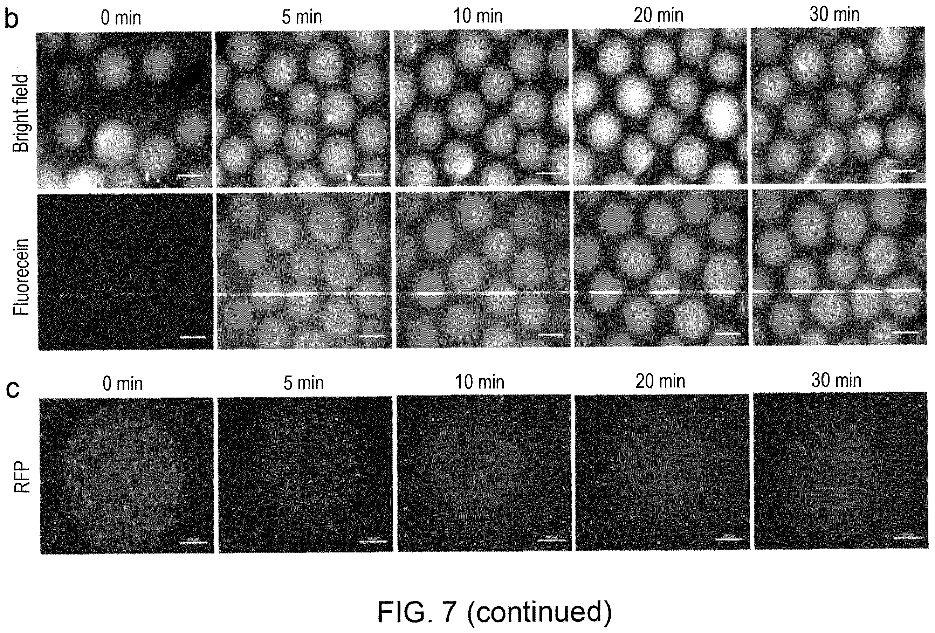

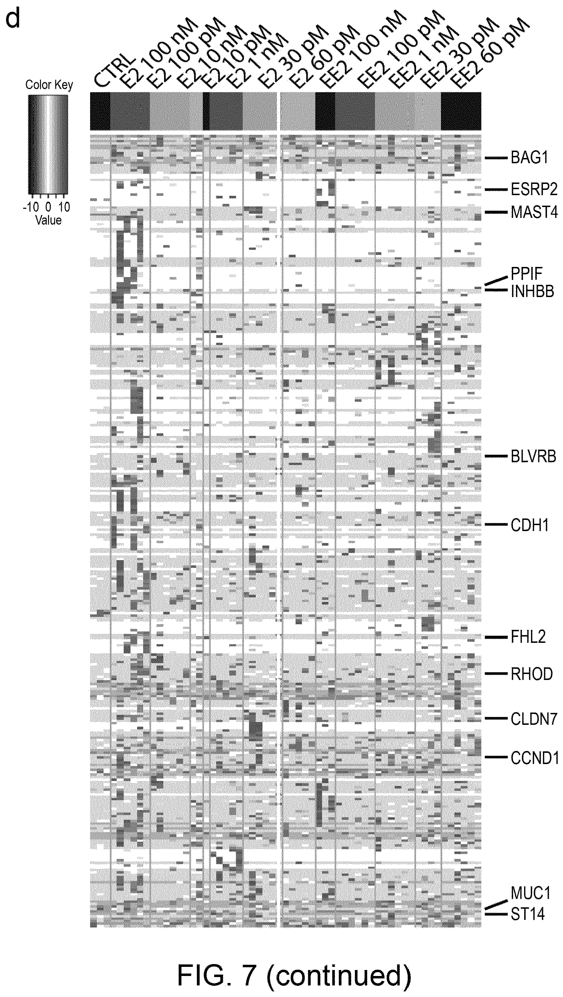

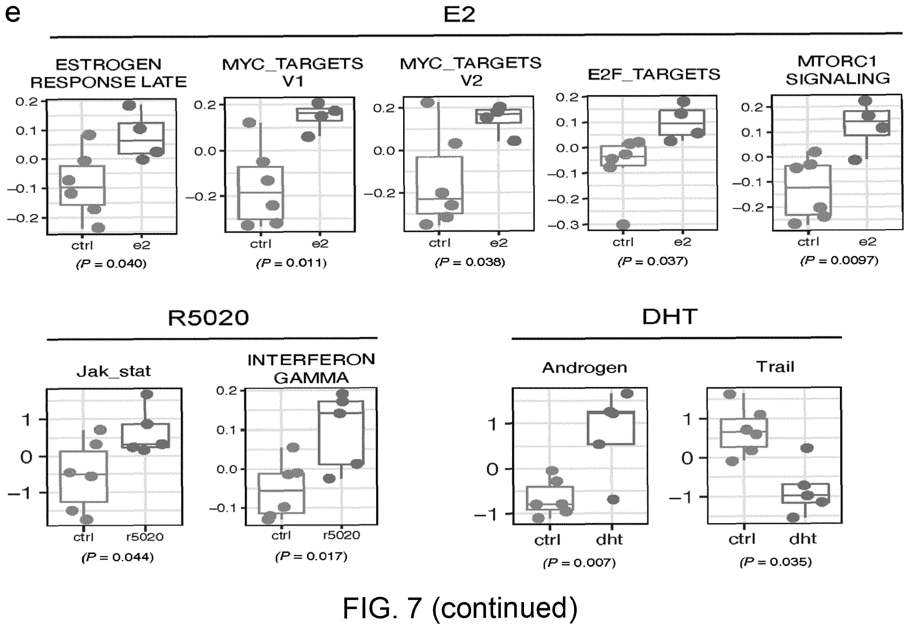

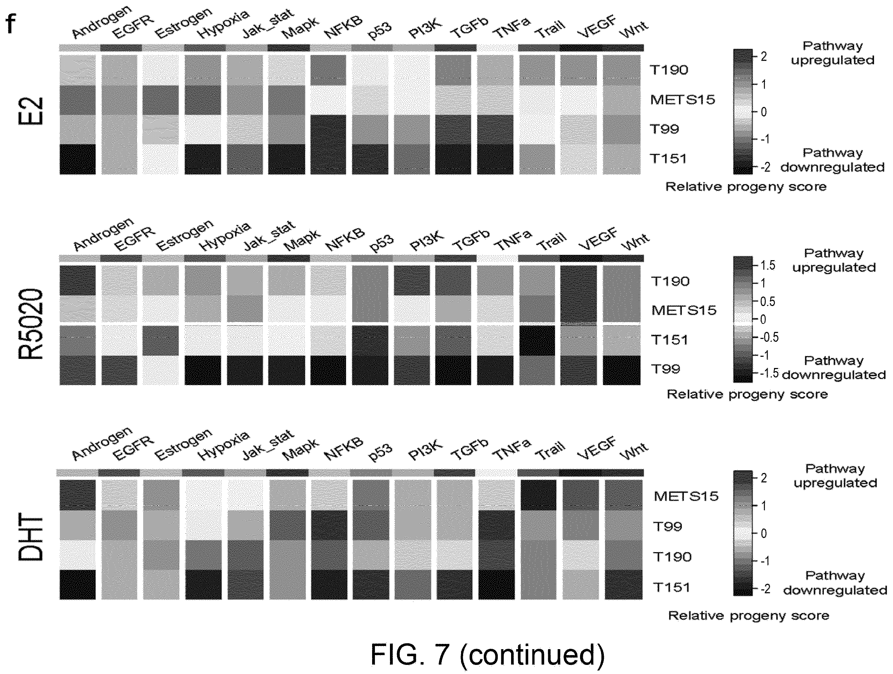

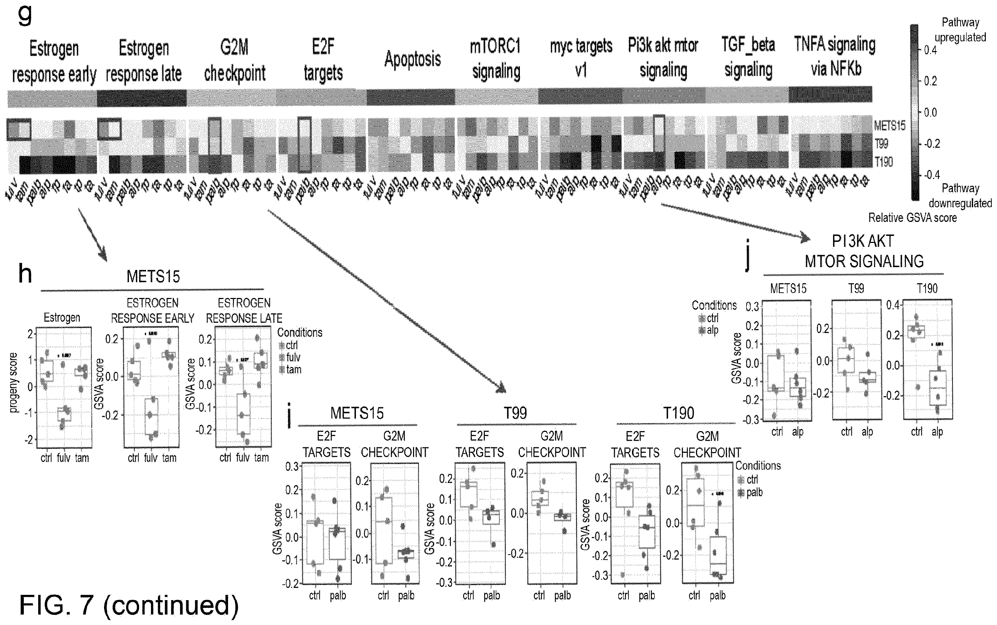

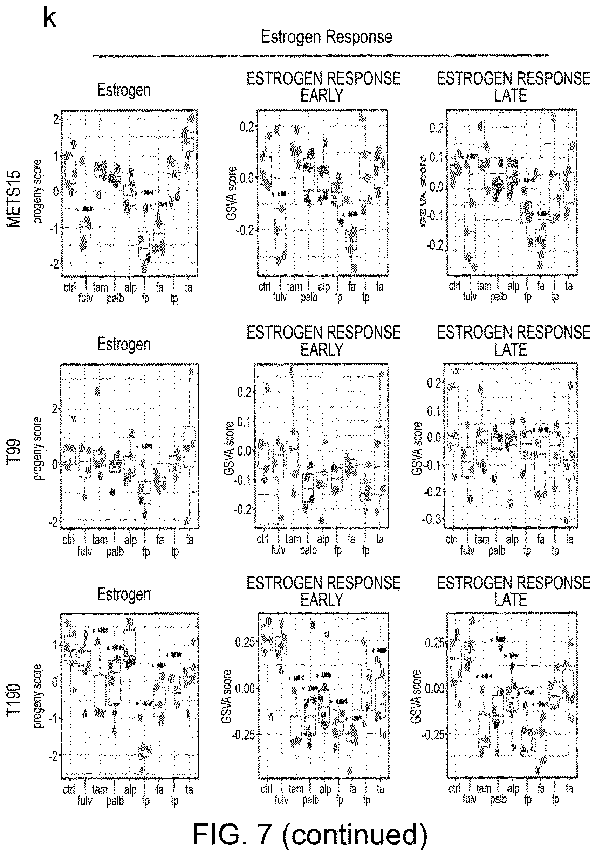

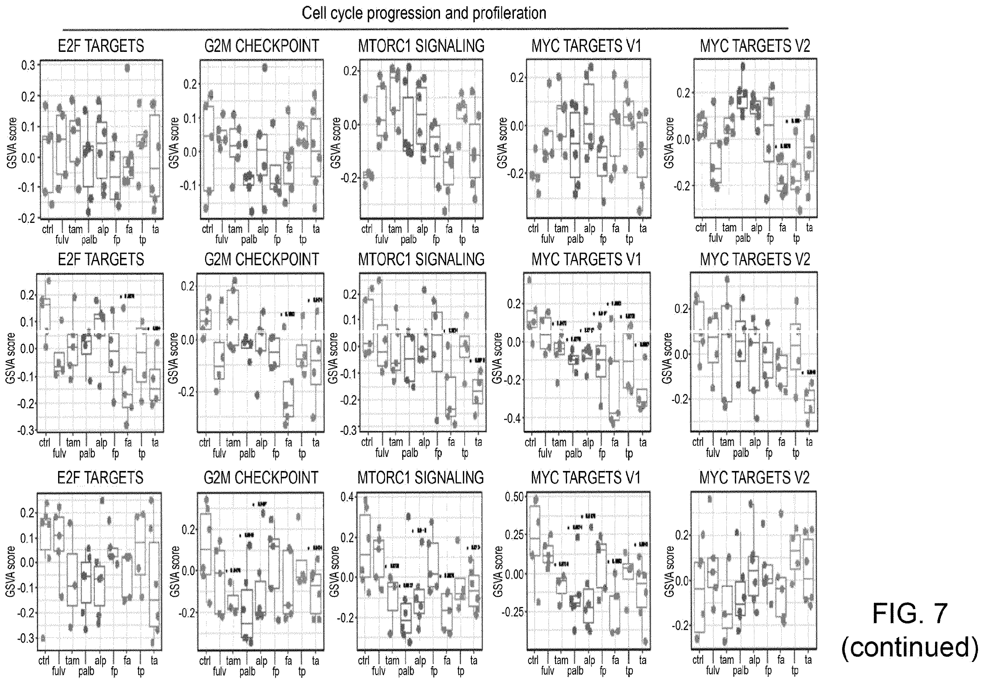

**Figure 7****. Automated workflow and RNA-seq results for hormone stimulation on primary normal breast tissue microstructures in alginate in miniatured 96 well plate screening format.**
   **a)** Illustration of automated workflow with tissue encapsulation in alginate, homogeneous dispensing of alginate into compound pre-coated 96 well plate and the plate plan with two estrogen receptor agonists, E2 and ethinylestradiol (EE2), with 7 concentrations for each compound and 6 technical replicates for each concentration, followed by principle of barcoding, one-step cell lysis, mRNA capture and reverse transcription when alginate is intact and not dissociated, NGS library preparation for RNA-seq analysis.
   **b)** Fluorescent micrographs showing sufficient diffusion of DNA barcodes modified with fluorescein into alginate beads in 10 minutes. Scale bars, 1 mm.
   **c)** Fluorescent micrographs showing complete cell lysis for RFP-expressing MCF7 cells in alginate in 20 min. Scale bars, 500 µm.
   **d)** Heatmap showing differentially expressed genes for normal breast tissue microstructures in alginate before and after E2 or EE2 treatment at 10 pM, 30 pM, 60 pM, 100 pM, 1 nM, 10 nM, 100 nM for 24 hours. ER target genes were successfully induced by both E2 and EE2 and were highlighted in texts on the right of heat map.
   **e)** Boxplots showing patient-derived breast tumor METS 15 responses to E2, R5020 and DHT after 24 h treatment in alginate *ex vivo* with representative significantly changed pathway selected. n = 4, 5, 6 as technical replicates.
   **f)** Heatmap showing pathway activities of breast tumor microstructures from 4 different patient-derived xenografts after 24 h treatment with ER agonist E2 (10 nM), PR agonist R5020 (10 nM) and AR agonist DHT (10 nM) in alginate *ex vivo.* Data represents PROGENy and GSVA pathway activity scores.
   **g)** Heatmap showing pathway activities of breast tumor microstructures from 3 different patient-derived xenografts after 24 h treatment with fulvestrant (100 nM), tamoxifen (200 nM), palbociclib (100 nM) and alpelisib (100 nM) in alginate *ex vivo.*
   **h)** Boxplots showing estrogen response of patient-derived breast tumor METS15 in responses to fulvestrant and tamoxifen after 24 h treatment in alginate *ex vivo.* Data represents z-score of PROGENy and GSVA pathway activities.
   **i)** Boxplots showing cell cycle progression determined by E2F targets and G2M checkpoint pathway activities in 3 different patient-derived breast tumor microstructures in response to CDK4/6 inhibitor palbociclib for 24 h in alginate *ex vivo.* Data represents PROGENy and GSVA pathway activity scores.
   **j)** Boxplots showing PI3K AKT MTOR signaling pathway acitivities of 3 different patient-derived breast tumor microstructures in response to Pi3K inhibitor alpelisib for 24 h in alginate ex vivo. Data represents PROGENy and GSVA pathway activity scores.
   **k)** Boxplots showing multiple pathway activities classified by estrogen response, cell cycle progression and proliferation. Data represents PROGENy and GSVA pathway activity scores.

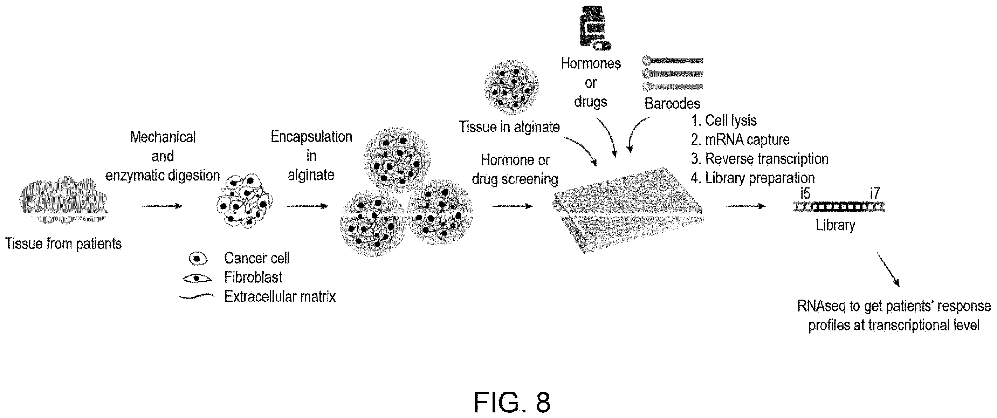

**Figure 8****: Scheme and design for alginate-based 3D ex vivo system to test hormone and drug sensitivity of breast tissue microstructures.**
   Collected patient tissue is dissociated into tissue microstructures where the proteins from ECM and cells from stroma (e.g. fibroblast) are preserved as a natural surround environment for the microstructures. The tissue microstructures are mixed with alginate and encapsulated in alginate crosslinked by Calcium ions. The alginate encapsulated breast tissue microstructures are suspended in phenol red-free and serum-free DMEM/F-12 excluding external sources of hormones and growth factors that can potentially stimulate breast tissue responses and treated with hormone receptor agonists or antagonists or drugs. At the end of treatment, mRNA from tissue microstructures is extracted for analysis of gene expression changes by RNA sequencing.

### Description

The present invention relates to a method for analyzing cellular responsiveness to hormones or drugs in a tissue sample from a patient or in a patient-derived tissue sample, comprising:
a) Mechanical and/or enzymatic digestion of said tissue sample to produce tissue microstructures;
b) Encapsulation of said tissue microstructures in alginate;
c) Exposing the encapsulated tissue microstructure to hormones or drugs;
d) Cell lysis, mRNA capture and reverse transcription in one-step
e) Alginate dissociation
f) RNA sequencing

Wherein step d) is performed with the alginate still crosslinked and intact.

Firstly, as part of the present method is an alginate-based 3D ex vivo physiologically relevant tissue culture system for normal and malignant tissue microstructures, such as breast tissue microstructure that can maintain the most important features of tissue, hormone receptors and hormone responses, in basic culture medium. The model overcomes the challenge of existing 3D ex vivo tissue culture system that fails to maintain hormone receptor expression and hormone response such as patient-derived organoids (PDOs) cultured in Matrigel^{®} or other 3D matrices. The present inventors demonstrated that tissue microstructures, such as breast tissue microstructures in alginate-maintained hormone receptors, hormone responses and proliferation in 7 days (see Example 1 and Fig. 1) and they responded to FDA-approved drugs on both transcriptional and translational level with response sensitivity in accordance with the potency of the drugs (see Example 3 and Fig.3): the down-regulation of target genes and proteins are stronger with more potent drugs. The present invention can faithfully recapitulate the hormone receptor status and hormone or drug responses of patients, and be used to guide therapeutic options in clinics.

Secondly, the inventors were able to miniaturize said alginate-based breast tissue microstructure system for high throughput transcriptomics-based compound screening with either commercially available plate dispenser or a microfluidics system. The key to establish this miniatured system was to integrate tissue lysis, mRNA capture and reverse transcription in one step, which involves fewer manual manipulations and is essential when using high throughput well plates with dispensers or using microfluidics chip with relatively closed environment that limits manual manipulation of reaction process. This one-step cell lysis, mRNA capture and reverse transcription reaction approach was never used in the prior art in complex system because tissue microstructure contains collagens and large cell debris that can inhibit the process, and the alginate is a potential barrier for cells to be exposed to reagents and thus can decrease the process efficiency. The inventors were able to overcome these obstacles by bypassing the normal alginate dissociation step which is routine in research and literature with alginate. Instead, said one-step cell lysis, mRNA capture and reverse transcription reaction approach was performed when alginate was still intact and crosslinked, and surprisingly, cDNA was harvested with good quantity and quality, in comparison, the inventors were not able to retrieve mRNA and synthesize cDNA when they used conventional way of alginate dissociation before cell lysis (see Example 6 and Fig. 6).

As such, tumor heterogeneity that faithfully recapitulates patient profile (especially the hormone receptor status and responses that are important for hormone sensitive cancers) is uniquely preserved in the form of 3D tumor microstructures in the present ex vivo drug screening method, even without intensive supplement of external hormones and growth factors which are required for maintaining the tissue growth ex vivo. Secondly, chemical and contextual information (e.g. stroma, extracellular matrix) of the tumor microenvironment are preserved. Thirdly, hormone receptor expression and signaling are preserved.

In one embodiment, the method for analyzing cellular responsiveness to hormones or drugs in a tissue sample from a patient or in a patient-derived tissue sample further comprises step g) performing one or more further assays and/or one or more further analytical techniques to determine the tissue responsiveness to hormones or drugs. Preferably, said further one or more assays or analytical techniques assess one or more of cell viability, cell proliferation, cell apoptosis, gene expression changes in the said tissue in response to hormones and drugs.

In one embodiment, the one or more assays or analytical techniques can be selected from MTT assay, Live/Dead staining, bioluminescence assay, immunofluorescent staining (IF), immunohistochemistry staining (IHC), western blot, polymerase chain reaction (PCR), RNA-seq and/or the one or more assays or analytical techniques can classify the said tissue sample as responsive or non-responsive to the hormones and/or drugs.

### Tissue sample

The present method can be used for analyzing cellular responsiveness to hormones or drugs in normal or malignant tissue sample from a patient. Preferably said tissue is a biopsy or tissue resection, from normal tissue and/or primary cancer and/or metastasis.

In one embodiment, said tissue sample is selected from the group consisting of epithelium tissue, connective tissue, cartilage tissue, bone tissue, muscle tissue, nerve tissue, blood vessel tissue, heart tissue, lymphatic system tissue, respiratory tract tissue, oesophagus tissue, urinary tract tissue, endocrine system tissue, reproductive system tissue, breast tissue and cancer tissue, preferably breast tissue, more preferably breast cancer tissue sample.

In a preferred embodiment, said tissue sample is an Estrogen Receptor positive (ER+) breast cancer tissue sample. In one embodiment, said the breast cancer patient has early-stage breast cancer or late-stage breast cancer.

### Mechanical and/or enzymatic digestion of said tissue sample to produce tissue microstructures

In the first step of the present method, tissue sample from a patient is mechanically and/or enzymatically digested to produce tissue microstructures, preferably mechanically and enzymatically digested. This allow to solve the issue of tissue samples being too large to feed the ex vivo assays.

In contrast with single cells, microstructures allow to preserve intact tissue architectures, hormone receptors, hormone responses and proliferation.

The digestion process can be any standard method known in the art. For example, https://onlinelibrary.wiley.com/doi/10.1002/cyto.a.23690⁴ *(accessed on 21*/*02*/*22)* summarized the best practice of solid tissue digestion and dissociation, and "https://www.worthington-biochem.com/tissuedissociation/default.html"⁵ *(accessed on 21*/*02*/*22)* summarized different digestion reagents, duration and other parameters for different tissue types.

In one embodiment, the tissue sample is mechanically dissociated with surgical scalpels or automated systems such as a vibratome or a tissue chopper to obtain pieces of 0,5 to 5 mm, preferably 1 to 5 mm of diameter or 1 to 30 mm³, preferably 1 to 15 mm³ of pieces.

In one embodiment, the tissue sample is mechanically dissociated and then incubated with enzymes selected from the group consisting of collagenase, hyaluronidase, benzonase, DNAse, neuraminidase, neutral protease, and mixtures thereof.

In preferred one embodiment, the tissue sample is mechanically dissociated and then incubated with 1% to 10%, preferably 1 to 5%, more preferably 1 to 2% by weight of collagenase A.

In one embodiment, digestion is performed in an incubator at 37 °C, in a humidified atmosphere preferably containing 5% CO2.

In one embodiment, the proteins from the extracellular matrix and cells from stroma (e.g. fibroblasts) are preserved as a natural surround environment for the microstructures.

In another embodiment, the tissue sample from a patient or the patient-derived tissue sample retains tissue heterogeneity and architecture, with proteins from the extracellular matrix and cells from stroma of the tissue sample preserved as a natural surround environment for the microstructures.

In one embodiment, the tissue sample maintains hormone receptor expression and functionality.

### Encapsulation of said tissue microstructures in alginate;

In the second step of the present method, microstructures produced in step a) are encapsulated in alginate.

The encapsulation process can be any standard method known in the art with commercially available encapsulators. For instance, Estrada MF et al.⁶ discloses a standard alginate microencapsulation method. Alginate is a natural and bioinert biomaterial. The present method is simpler and cleaner than standard method comprising collagen-based extracellular matrix such as hydrogel.

In one embodiment, no collagen-based biomaterial is added during the encapsulation.

In one embodiment, the tissue microstructures are mixed with alginate and encapsulated in alginate crosslinked by calcium ions.

In one embodiment, the alginate encapsulated tissue microstructures are suspended in phenol red-free and serum-free basal medium such as DMEM/F-12.

In one preferred embodiment, no additional hormones and/or growth factors are added during the encapsulation or culture as they can potentially stimulate tissue responses and disrupt results.

In one embodiment, the microstructure encapsulation is conducted on an electrostatic bead generator. A voltage can be applied between the needle of the nozzle and the surface of gelling bath to decrease the size of alginate beads. The size of alginate beads can be tuned by changing the voltage, needle size of the nozzle, distance between the needle and the surface of gelling bath, and combinations thereof.

In one embodiment, alginate beads are preferably washed at least once with NaCl and transferred to phenol red-free and serum-free basal medium preferably containing 1% penicillin/streptavidin and preferably 0.1 mg/mL of a broad-spectrum antibiotic formulation designed to protect primary cells from microbial contaminations such as Primocin^{®} for standard culture in an incubator.

In one embodiment, the tissue microstructures are encapsulated in alginate beads having a diameter of between 10 to 1200 µm, preferably between 40 to 800 µm, more preferably between 50 to 500 µm.

In one embodiment, encapsulated tissue microstructures are maintained in culture for 3h to 23 days, preferably 5h to 14 days, more preferably 12h to 10 days, even more preferably 24h to 7 days.

In one embodiment, encapsulated tissue microstructures are in static culture or cultured without agitation, which can apply too much shear stress to the tissue and does not represent physiologically relevant environment for tissue in vivo.

### Exposing the encapsulated tissue microstructure to hormones or drugs

After encapsulation of the tissue microstructures in alginate, said encapsulated microstructures are exposed to hormones or drugs.

Said hormones or drugs can be any compound of interest for hormone and drug testing for personalized medicine, drug discovery, drug development, or pre-clinical studies.

In one embodiment, said hormones are selected from the group consisting of receptor agonists or receptor antagonists, preferably estrogen receptor agonist or antagonist, progesterone receptor agonist or antagonist, androgen receptor agonist or antagonist and glucocorticoid receptor agonist or antagonist.

In one embodiment, said hormones are selected from the group consisting of E2 (estrogen receptor agonist), EE2 (ethinyl estradiol, synthetic estrogen receptor agonist), progesterone (progesterone receptor agonist), R5020 (progesterone receptor agonist), DHT (androgen receptor agonist), dexamethasone (glucocorticoid receptor agonist), and progestins such as levonorgestrel, chlormadinone acetate, desogestrel, cyproterone acetate, gestodene and drospirenone.

In one embodiment, said drugs are selected from the group consisting of FDA-approved drugs and new drugs under clinical trials.

In one embodiment, said drugs are selected from the group consisting of tamoxifen, fulvestrant, mifepristone, enzalutamide, palbociclib, and alpelisib.

### One-step tissue lysis, mRNA capture and reverse transcription

In the present method, it is needed to isolate mRNA for downstream RNA sequencing to allow transcriptomics-based readout, although tissue microstructures, such as breast tissue microstructures in alginate poses challenge in mRNA isolation.

Tissue microstructures have more complex biochemical and cellular components (proteins from the extracellular matrix (ECM), such as collagens, elastins, fibronectins, laminins, proteoglycans, etc, and different cell types in stroma) than the single cells that can be from either cell lines or digested patient samples. This would not be an issue when using RNA isolation with traditional Trizol^{®} method where the harsh reagent Trizol^{®} can denature almost all the components and will not leave any cell debris or residual proteins/DNAs that may affect downstream process (determined by the method principle with silica membrane column), however, Trizol^{®}-based RNA extraction requires intensive manual work and is not suitable for high throughput screening or for the adaption of the workflow to microfluidics devices.

To overcome these challenges, the present inventors surprisingly found that, in contrast with the conventional processing sequence, where the alginate is dissolved first to expose cells and cells are lysed later, it was possible to take advantage of the nature of crosslinked alginate which is highly permeable (reagents can diffuse in to lyse the cells and initiate reverse transcription) and porous (adequate space for mRNA release and cDNA synthesis), by lysing the cells and synthesize cDNA while the alginate is still crosslinked and intact. The alginate could then be dissolved afterwards to retrieve all the cDNA. In this way, the inhibitory effect from cells debris (which are immobilized and confined in alginate during reverse transcription) and from the dissolved alginate are avoided. Other strategies were tested by the inventors and failed to synthesize cDNA (see example 6 and Fig. 6), such as no dissociation of alginate before and after cell lysis and RT, showing that dissociation of alginate after RT is crucial to free all the cDNA from the alginate (shown in Fig. 6c), separating cell lysis and RT into two independent processes also failed to work (shown in Fig. 6d). This shows the relevance of the step sequences of the present invention.

As such, in the present method, after the encapsulated tissue microstructure have been exposed to hormones or drugs, tissue lysis, mRNA capture and reverse transcription are performed in one step while the alginate is still crosslinked and intact (i.e no alginate dissociation step has been performed before this one-step approach).

As used herein "in one step" means that the cell lysis, mRNA capture and reverse transcription are performed simultaneously in the same chamber or well.

In other words, a cell lysis buffer is combined with RT mix in the same chamber or well that has unique DNA barcode for different treatments and is followed by a standard reverse transcription incubation. This allows the cell lysis, mRNA capture and the RT step to be performed in a single reaction.

As used herein, "well" or "chamber" refers to any structure with the capacity to hold a sample sufficient to perform the methods described herein. One of skill in the art will know that a "well" or a " chamber " can include, e.g., a recess in a plate, a micro tube, a spot on a glass slide created by surface tension, a region of a microfluidic device or a droplet generated by microfluidic device.

Any commercially available lysis buffer can be used in the present method, for instance IGEPAL^{®} CA-630. Any suitable RT mix or enzyme can be used in the present method, for instance Maxima-H RT.

The mRNA capture strategy can be any conventional strategy, such as capture probes with poly-dT tail.

In one embodiment, the mRNA capture agent is a bead, comprising immobilized oligonucleotides. In one aspect, the bead is a silica bead, hydrogel bead or a magnetic bead. The mRNA capture agent comprises oligonucleotides which hybridize mRNA; in particular, the oligonucleotides comprise at least one poly(T).

In one embodiment, the mRNA capture agent is biotin that can be captured by specific binding to Streptavidin-coated magnetic beads. This allow the mRNA to be "fished out" and purified Because of its solubility, Biotin is advantageous to avoid clogging when using small tubings or microfluidics channels.

In one embodiment, the mRNA capture can be coupled with molecular barcoding.

Molecular barcoding generally involves attaching (for example, by ligation or by primer extension) a unique nucleic acid label (a 'barcode') to several single target molecules (DNA or RNA) in a solution containing a large number of such molecules. These labelled molecules are then sequenced, which for each reveal both the sequence of the molecular barcode, and at least part of the sequence of the labelled target molecule itself.

For instance, in one embodiment the present inventors included a ligation process for two barcodes in the cell lysis/RT mix where the poly-T tail of the barcode can capture the poly-A tail of the mRNA. Single strand DNA barcode with poly-T tail can also be used as an alternative form of barcode. (I also added single strand DNA barcode sequence in materials table 2)

### Alginate dissociation

Once the previous "one-step" approach achieved, the alginate is dissolved in a step of alginate dissociation (or alginate dissolution) to retrieve all the cDNA (in contrast with conventional methods where the alginate is dissolved before the cell lysis, mRNA capture and reverse transcription).

In this way, it allows to avoid the inhibitory effect from cellular debris (which is immobilized and confined in alginate during reverse transcription) and from the dissolved alginate.

Any conventional alginate dissociation buffer can be used in the present method, for instance one comprising sodium citrate and sodium chloride.

### High-throughput sequencing to get patients' response profiles at transcriptional level

The last step of the present method is the analysis of gene expression changes by RNA-Seq (named as an abbreviation of RNA sequencing) to get patients' response profiles at transcriptional level.

RNA-Seq is a very well-known sequencing technique which uses High-throughput sequencing (also called next-generation sequencing (NGS)) to reveal the presence and quantity of RNA in a biological sample at a given moment, analyzing the continuously changing cellular transcriptome.

The RNA-Seq step can be performed according to any standard method known in the art. The RNA-Seq improves the prediction of drug effectiveness according to the up- or down-regulation of target genes.

In one embodiment, DNA barcoding strategy is incorporated into the system to improve the throughput/screening capacity of the system.

Since the present method faithfully reflect original patient samples (especially the hormone receptor status, responses, expression and signaling that are important for hormone sensitive cancers), preserve chemical and contextual information (e.g. stroma, extracellular matrix) of the tumor microenvironment, the RNA sequencing can provide reliable patient-specific response profiles and/or prediction of drug effectiveness according to the up-or down-regulation of target genes.

### Method Applications

The present method can be used for hormone and drug testing on patient sample for personalized medicine, drug discovery, drug development, pre-clinical or clinical studies.

For instance, starting from a patient sample, with the present method it is possible to generate hundreds to thousands of alginate beads and exposing them to e.g., a set of FDA-approved drugs and combinations thereof and thus perform a fast screening while maintaining hormone response that is essential to make valid predictions on optimal therapies.

For patients that are treatment-naïve, the present method can be used in drug screening with approved drugs and combinations, to guide clinicians for decision-making on which therapeutic options to give to patients.

For patients that are not responding or resistant to any available routine treatments, the present method can be used for drug screening with approved drugs but in unusual combinations, or with new drugs under clinical trials, to help prolong the survival of patient as much as possible.

The present method can be also used for individuals who are interested in understanding their own responses to hormones and drugs.

### EXAMPLES

The foregoing descriptions will be more fully understood with reference to the following Examples. Such Examples, are, however, exemplary of methods of practicing the present invention and are not intended to limit the scope of the invention.

### Materials and method

### Cell culture

Estrogen receptor positive cell lines MCF7, HCC1428, T47D and MDA-MB-134 were purchased from the American Type Culture Collection (ATCC). All cell lines except MDA-MB-134 were cultivated in Dulbecco's modified Eagle's medium (DMEM) medium (cat# 31966, Gibco) with 10% FBS (Corning) and 1% penicillin/streptomycin (cat# 15070-063, Thermo Fisher Scientific Inc.). All cell lines were maintained in humidified incubator supplemented with 5% CO2 at 37 °C. Mycoplasma test was performed routinely. Lentivirus production and purification were performed according to protocols in publications (Barde et al., 2010; Tiscornia et al., 2006) and used to transduce breast cancer cell lines to express firefly luciferase (luc2) under the control of CMV promoter (vector pCDH-CMV-MCS-EF1-puro-luc2) and DsRed fluorescent protein by hPGK-DsRed. Brightest DsRed subpopulation were selected by FACS sorting.

### Mice

All mice were maintained and handled according to Swiss guidelines for animal safety, with a 12-h-light-12-h-dark cycle, and controlled temperature and food and water ad libitum. Experiments were performed in accordance with protocols approved by the Service de la Consommation et des Affaires Vétérinaires of Canton de Vaud, Switzerland. NOD.Cg-Prkdcscid Il2rgtm1Wj1/SzJ mice (NSG) were purchased from Jackson Laboratories.

### Transplantation and intraductal injection

For transplantation, engrafted mammary glands were mechanically dissociated into fragments with 1 mm³ volume and then enzymatically digested with 1.5% v/v collagenase A (Roche) in DMEM/F12 phenol red free medium (Thermo Fisher Scientific Inc.) containing 1% penicillin/streptomycin on a tube roller with speed of 45 rpm at 37 °C for 1 h. Cells were dissociated to single cells with 0.25% trypsin-EDTA (Gibco, 15400), resuspended with red cell blood lysis buffer, and passed through 40 µm cell strainer. To isolate human cells from humanized mammary glands, single cells were incubated with mouse cell depletion cocktail (Miltenyi Biotec) and passed through LS columns (130-042-401) on MACS separator according to manufacturer's protocol (Miltenyi Biotec).

For cell line injection, MCF7 cells were trypsinized with 0.25% trypsin-EDTA and resuspended in DMEM with 10% FBS and 1% penicillin/streptomycin, followed by filtration through a 40 um cell strainer. Cells with size ranging from 7-20 um were counted and desired amounts of cells were resuspended in PBS for injection. Female NSG mice were anaesthetised by intraperitoneal injection with 10 mg/kg xylazine and 90 mg/kg ketamine (Graeub). Single cell suspensions were injected intraductally through cleaved teats using Hamilton syringes, with 1×105 - 2×105 cells per gland from cell lines for transplantation.

### Bioluminescence imaging

To monitor the growth of tumor, mice were intraperitoneally injected with 150 mg/kg luciferin (cat# L-8220, Biosynth AG) and imaged with Xenogen IVIS Imaging System 200 (Caliper Life Sciences) 12 min after injection. Mice were monitored every two weeks for the first 5 month and then monthly. Images were acquired and analyzed with Living Image software (Caliper Life Sciences, Inc.).

### Tissue dissociation and encapsulation with commercially available encapsulator

Breast carcinoma from mammary gland xenografts were mechanically dissociated into 1 - 30 mm³ of microstructures with surgical scalpels, followed by enzymatic digestion in phenol red free DMEM/F-12 containing 1% penicillin/streptavidin and 1.5% of Collagenase A on a tube roller at 45 rpm at 37 °C for 1 h. After centrifugation at 2200 rpm for 4 min at 4°C, the pellet was resuspended in 1 mL of DNase (1 mg/mL, Sigma-Aldrich) for 3 min at room temperature, followed by centrifugation and resuspension in 3 mL of red blood cell lysis buffer (Sigma-Aldrich) for 5 min at room temperature. Pellets dissociated from every 1 g of mammary gland were dispersed in 10 mL of 1% (w/v) of Ultrapure Ca2+ MVG alginate (UP MVG NovaMatrix, Pronova Biomedical) dissolved in 0.9% (w/v) of NaCl. The microstructure encapsulation was conducted on an electrostatic bead generator (Nisco Var V1) with a nozzle 0.9 mm in diameter. A voltage was applied between the needle of the nozzle and the surface of gelling bath to decrease the size of alginate beads. Cross linking process was triggered by calcium ions in the gelling bath containing 100 mM calcium chloride (Sigma-Aldrich) and 10 mM HEPES (Sigma-Aldrich) pH 7.4 for 10 min, and the produced alginate beads result in an average size of 1 mm in diameter. Alginate beads were washed with 0.9% NaCl for 3 times and transferred to phenol red free DMEM/F-12 containing 1% penicillin/streptavidin for standard culture in the incubator with 5% CO2 at 37 °C.

### qPCR

Messenger RNA was isolated from tissue microstructures in 1% of alginate and converted into cDNA by reverse transcription. Real time qPCR reactions were performed in triplicates with SYBR Green FastMix (Quantabio) using 7900HT qPCR instrument (Applied Biosystems). Primers used for qPCR are shown in Table 1.

### Immunohistochemical assay

Microstructures encapsulated in alginate beads were fixed in 4% paraformaldehyde supplemented with sucrose at 4 °C overnight and embedded in paraffin. Sections with 4 µm in thickness were stained with hematoxylin and eosin according to standard protocols. For immunohistochemical staining, sections were dewaxed, rehydrated and processed to antigen retrieval with 10 mM trisodium citrate buffer, pH 6.0 for 20 min at 95 °C. After blocking with 1% BSA for 1 h at room temperature, the sections were incubated with primary antibodies diluted in 1% BSA at 4 °C overnight, followed by secondary antibodies incubation for 1 h at room temperature. Nuclei were counterstained with Fluoromount-gel containing DAPI (Invitrogen).

### Fabrication of microfluidics chips

Microfluidics chips were prepared by photolithography in the Center of MicroNanoTechnology (CMi) at EPFL. The design of microfluidic channels was patterned on silicon wafer by direct writing with VPG200 (Heidelberg Instruments) and dry etched by Bosch process with AMS200 deep reactive ion etching (Alcatel, France) to reach a channel height of 150 um. The remaining photoresist was stripped in O2 plasma (TePla), followed by silanization of the silicon wafer with trichloro (1H,1H,2H,2H-perfluorooctyl) silane (Sigma-Aldrich). PDMS and curing agent with ratio of 10:1 was mixed, defoamed and degassed before mounting onto the silicon wafer with curing process of 80 C for 2 hours. PDMS was unmolded, cut, punched to create inlets/outlets, exposed to O2 plasma and bonded to glass slides to form final microfluidics chips.

### Cell encapsulation on microfluidic chips

Breast tissue microstructures or cell lines were trypsinized, centrifuged and resuspended in the calcium-EDTA/alginate mixture (1 w% alginate, 50 mM Ca-EDTA) and loaded into polycarbonate syringe (BD Luer-Lok disposable syringes) that connected to PE/2 tubing with the other end inserted in the chip inlet for aqueous phase. Perfluorinated carbon oil (3M Novec 7300 Engineered Fluid) containing 0.05% (v/v) acetic acid was loaded in polycarbonate syringe and introduced in chip inlet for oil phase, where the acetic acid triggered the release of calcium ions to induce crosslinking of alginate droplets. A washing and extraction step was then performed with 20% (v/v) solution of perfluoro-1-octanol (PFO, Sigma-Aldrich) in perfluorinated carbon oil to break emulsion and extract alginate beads from oil phase into aqueous phase. Then, Alginate beads were centrifuged and resuspended in culture medium. LIVE/DEAD^{®} cell viability assay (Thermo Fisher) and fluorescence microscopy were used to determine cell viability.

### Automated homogeneous dispensing of tissue microstructures in alginate in 96 well plate

Breast tissue microstructures were encapsulated in alginate and resuspended in DMEMF-12 medium supplemented with anti-fungus Primocin and 1% P/S in 50 mL centrifuge tube. The tube was then mounted on the MultiFlo^{™} FX Multi-Mode Dispenser (Biotek) and alginate beads suspension was aspirated by the peri-pump and dispensed through dispense cassettes (Biotek or Steinle Labtechnology) into 96 well plate pre-coated with compound nanodroplets prepared by acoustic liquid handler Labcyte Echo 550 (Beckman Coulter) at Biomolecular Screening Facility at EPFL. Each well was covered with 20-40 beads with a final volume of 100 uL that results in working concentration for each compound. The combination of dispenser and compound pre-coated 96 well plate significantly increased the alginate beads distribution efficiency with reduced hand-on time from 4 hours to 5 min.

### Cell lysis, mRNA capture and Reverse Transcription in alginate

At the end point of compound treatment, alginate beads with medium were transferred to 96-well PCR plate and sedimented for 5 min to allow alginate settle down at the bottom of wells. Medium was then removed from 96 well plate with MultiFlo^{™} FX Multi-Mode Dispenser with aspiration mode and alginate beads were left at the bottom of the wells. Lysis and RT mix were added directly into each well when the alginate is intact and not dissociated, where the RT mix contained 0.5x Maxima RT buffer, 0.1-0.5% IGEPAL (Sigma-Aldrich), 0.5 mM dNTPs (Life technology), 2 U/µL Rnase Inhibitor (Lucigen), 2.5 µM Template_Switch Oligo (Table S1), 10 U/µL Maxima H Minus Reverse Transcriptase (ThermoScientific), 1x T4 ligation buffer (NEB), 20,000 U/mL T4 ligase (NEB), Barcode-biotin (Table S1) and Barcode-dT (Table S1). The alginate beads were incubated with lysis and RT mix at room temperature for 30 minutes to allow for cell lysis, barcode ligation and mRNA capture, followed by 42 C for 90 minutes for reverse transcription. To retrieve all the cDNA inside the alginate, alginate dissociation buffer containing 50 mM of sodium citrate and 104 mM of sodium chloride was then added into each well and incubated at 37 C for 20 min. Dynabeads^{™} MyOne^{™} Streptavidin C1 (ThermoScientific) were used to separate mRNA by specific binding between streptavidin and biotinylated barcode that captures mRNA with poly-dT tail on a magnetic stand MagRack 6 (VWR International) or a magnetic separation plates 96 well side full bar Magnet PCR separation plate (Labgene Scientific). The captured mRNA were then washed twice with 1x TE + 0.5% Sodium Dodecyl Sulfate (TE/SDS, Sigma-Aldrich) and twice with nuclease free water (Sigma-Aldrich). MSeI restriction Enzyme (NEB) was used to cleave the barcoded mRNA off the streptavidin C1 beads. Released DNA fragments were size selected by purification using 0.6x Agencourt AMPure XP beads beads (Beckman Coulter) according to manufacturer's instructions and were amplified by PCR in a volume of 50 µL using 1x Hifi HotStart Readymix (Roche) and 0.6 µM Template_Switch_PCR primer (Table S1). The aliquots were thermocycled as follows: 95 C 3 min; then four cycles of: 98 C for 20 sec. 65 C for 45 sec, 72 C for 3 min; then 15 cycles of: 98 C for 20 sec, 67 C for 20 sec, 72 C for 3 min; then a final extension step of 5 min at 72 C. The products were then purified with 0.6x Agencourt AMPure XP beads according to the manufacturer's instructions and eluted in 10 µL of H2O. The concentration of the cDNA was measured on Invitrogen^{™} Qubit^{™} 3 (Invitrogen) and the cDNA profile was analyzed on a Fragment analyzer (Agilent Technologies).

### Preparation of cDNA Library for Sequencing

To prepare 3'-end cDNA fragments for sequencing, 400 pg of cDNA were used as input in Nextera XT tagmentation reactions with Tn5 enzyme produced by Protein Production and Structure Core Facility at EPFL. Custom primers P5_TSO_Hybrid and Nextera_N70X (Table 1) were used in the PCR mix for amplification as follows: 95 C for 3 min; 12 cycles of 98 C for 20 sec, 62 C for 15 sec, 72 C for 30 sec; then a final extension step of 72 C for 3 min. The PCR product was purified using Agencourt AMPure XP Beads according to the manufacturer's instructions, and eluted in 10 µL of water. The concentration of elution was determined by Qubit and the profile was analyzed using Fragment analyzer. The average size of sequenced libraries was between 350 and 650 bp.

The libraries were sequenced on the Illumina NextSeq 500 with Read1CustSeqB (Table 1 below) for priming of read 1 at Gene Expression Core Facility at EPFL. Read 1 was 25 bp (barcode); read 2 was 50 bp (paired end).

**Table 1**

| **Primers** | **Forward and reverse sequences** |
|---|---|
| *HPRT* | 5'-GACCAGTCAACAGGGGACAT-3' |
| | 5'-CCTGACCAAGGAAAGCAAAG-3' |
| *GAPDH* | 5'-AGGGCTGCTTTTAACTCTGGT-3' |
| | 5'-CCCCACTTGATTTTGGAGGGA-3' |
| *ESR1* | 5'-GGAGATCTTCGACATGCTGC-3' |
| | 5'-GCCATCAGGTGGATCAAAGT-3' |
| *PGR* | 5'-GCCATCAGGTGGATCAAAGT-3' |
| | 5'-CGTAGCCCTTCCAAAGGAAT-3' |
| *GREB1* | 5'-CAGCTGACTGTCTTCCACCA-3' |
| | 5'-AGCCCTGAAGTGTTTTGCTG-3' |
| *AR* | 5'-CCCACTTGTGTCAAAAGCGA-3" |
| | 5'-AATGGGCAAAACATGGTCCC-3' |
| *TMPRSS2* | 5'-CCATTTGCAGGATCTGTCTG-3' |
| | 5'-GGATGTGTCTTGGGGAGCAA-3' |
| *Ki67* | 5'-CGGACTTTGGGTGCGACTT-3' |
| | 5'-GTCGACCCCGCTCCTTTT-3' |
| *FOXM1* | 5'-TCTGCCAATGGCAAGGTCTCCT-3' |
| | 5'-CTGGATTCGGTCGTTTCTGCTG-3' |
| *BCL2* | 5'-GGTGGGGTCATGTGTGTGG-3' |
| | 5'-CGGTTCAGGTACTCAGTCATCC-3' |

**Table 2**

| | |
|---|---|
| Barcode-biotin | [BIO]TTTTTTTAAGCAGTGGTATCAACGCAGAGTACNNNNNNNNNNgcggc |
| | [Pho]NNNNNNNNNNGTACTCTGCGTTGATACCACTGCTTAAAAAAA |
| Barcode-dT | [Pho]NNNNNNNNNNTTTTTTTTTTTTTTTTVN NNNNNNNNNNgccgc |
| Barcode-single | [BIO]AAGCAGTGGTATCAACGCAGAGTACNNNNNNNNNNNNNNNNVVTTTTTTT TTTTTTTTTTTTTTTTTTTTTTTTVN |
| Template_Switch _PCR primer | AAGCAGTGGTATCAACGCAGAGT |
| Template_Switch _Oligo | AAGCAGTGGTATCAACGCAGAGTGAATrGrGrG |
| P5_TSO_Hybrid | AATGATACGGCGACCACCGAGATCTACACGCCTGTCCGCGGAAGCAGTGGT ATCAACGCAGAGT*A*C |
| Nextera _N701 | CAAGCAGAAGACGGCATACGAGATTCGCCTTAGTCTCGTGGGCTCGG |
| Nextera _N702 | CAAGCAGAAGACGGCATACGAGATCTAGTACGGTCTCGTGGGCTCGG |
| Nextera _N703 | CAAGCAGAAGACGGCATACGAGATTTCTGCCTGTCTCGTGGGCTCGG |
| Nextera _N704 | CAAGCAGAAGACGGCATACGAGATGCTCAGGAGTCTCGTGGGCTCGG |
| Read1CustSeqB | GCCTGTCCGCGGAAGCAGTGGTATCAACGCAGAGTAC |

### Example 1: Alginate encapsulated breast tumor microstructures preserves hormone receptor expression and maintain cell proliferation for 7 days ex vivo.

The inventors demonstrated that according to the present invention, alginate encapsulated breast tumor microstructures preserves hormone receptor expression and maintain cell proliferation for 7 days ex vivo (see Fig 1).

Fluorescent Immunohistochemistry staining was performed (see Fig. 1a) on alginate encapsulated patient derived tumor microstructures on 4 µm paraffin-embedded sections for hormone receptors (ER and PR), proliferation markers (Ki67 and pHH3) and apoptosis marker (Cleaved caspase 3). Sections were labeled with human specific E-cadherin to identify patient cells and stained with DAPI to detect cell nuclei. The cells positive for human specific proliferation and apoptosis markers as well as hormone receptors over total numbers of cells stained with DAPI were then quantified (see Fig. 1b).

Fluorescent Immunohistochemistry staining was performed (see Fig. 1c) on sections from patient derived tumor (mouse mammary gland injected with the same patient cells) labeled with human specific E-cadherin to identify patient cells and stained with DAPI to detect cell nuclei. Cells positive for human specific ER and PR over total numbers of cells stained with DAPI were then quantified to indicate hormone receptors and proliferation levels (see Fig. 1d). The observation that the percentage of PR+ cells was higher than that of ER+ cells reflected the hormone status of its clinical origin, where percentage of PR was higher than that of ER when the patient was diagnosed. Also, ER, PR, Ki67 and pHH3 expressions in patient derived tumors were comparable to patient derived tumor microstructures encapsulated in alginate, indicating that alginate-based system can faithfully preserve the characteristics of patient derived tumors.

### Example 2: Tumor microstructures in alginate respond to both hormone receptor agonist E2 at transcriptional level, demonstrating the functionality of hormone receptors ex vivo.

The inventors demonstrated that tumor microstructures in alginate respond to hormone receptor agonist E2, at transcriptional level, demonstrating the functionality of hormone receptors ex vivo by analyzing the genes expression (see Fig. 2a) and performing a pathway enrichment analysis (see Fig. 2b) of patient derived tumor microstructures with large and medium sizes encapsulated in alginate, before and after 10 nM E2 treatment for 24 hours.

### Example 3: Tumor microstructures in alginate respond to selective ER degrader (fulvestrant) and selective ER modulator (tamoxifen) during 7 days at both transcriptional and protein levels.

The inventors demonstrated that tumor microstructures in alginate respond to selective ER degrader (fulvestrant) and selective ER modulator (tamoxifen) during 7 days at both transcriptional and protein levels.

The relative transcriptional levels of ER target genes, *ESR1*, *PGR*, *GREB1*, and proliferation marker *Ki67* upon 100 nM fulvestrant and 200 nM tamoxifen treatment for microstructures in alginate during 7 days were quantified (see Fig. 3 a).

Fluorescent Immunohistochemistry staining was performed (see Fig. 3 b) on microstructures in alginate treated with 100 nM fulvestrant or 200 nM tamoxifen for 3 days for human-specific E-cadherin and ER, PR, Ki67 and cleaved caspase 3 counterstained with DAPI (blue). The human-specific ER+, PR+, Ki67+ and cCasp3+ cells over total numbers of cells stained with DAPI from said staining were quantified (see Fig 3 c).

### Example 4: Alginate encapsulated primary normal breast tissue microstructures preserves hormone receptor expression and maintain cell proliferation for 23 days ex vivo.

The inventors demonstrated that according to the present invention, alginate encapsulated primary normal breast tissue microstructures preserves hormone receptor expression and maintain cell proliferation for 23 days ex vivo. Primary normal breast tissue microstructures were cultured in alginate for 1, 3-, 7-, 14- and 23-days ex vivo (see Fig. 4 a). Immunofluorescent staining of alginate encapsulated primary normal breast tissue microstructures was performed on 4 µm paraffin-embedded sections with hormone receptors (ER and PR), proliferation marker (Ki67) and apoptosis marker (Cleaved caspase 3). Sections were labeled with human specific E-cadherin and stained with DAPI to detect cell nuclei. (see Fig. 4 b).

Percentage of cells positive for hormone receptors, proliferation and apoptosis markers over total numbers of cells stained with DAPI was performed (see Fig. 4 c). No significant decrease was detected in hormone receptor expression and proliferation of normal tissue microstructures in alginate over 23 days culture ex vivo. Fluorescent Immunohistochemistry staining was performed for ER and PR counterstained with DAPI of primary normal breast tissue microstructures in alginate treated with 10 nM E2 for 3 days (see Fig. 4 d). Primary normal breast tissue responded to E2 stimulation with down-regulation of ER protein and up-regulation of PR protein expressions.

Percentage of cells positive for ER and PR over total numbers of cells stained with DAPI was performed to indicate hormone receptors and proliferation levels (see Fig. 4 e).

### Example 5: Alginate beads containing cells can be generated on microfluidic chip with high size and shape uniformity and high cell viability.

The inventors were able to perform a microfluidic preparation of cell-containing alginate beads generated at the flow focusing geometry and crosslinked in the delay channel with released calcium ions from EDTA-Ca (see Fig. 5 a). Flow rate is 1500 µL/h for oil phase and 300 µL/h for alginate phase.

Micrograph and size distribution of alginate beads showed an average diameter of 195 µm and high size uniformity (see Fig. 5b).

Cell viability was determined by live/dead (Calcein-AM/Ethidium homodimer) staining at 2, 5, 10 min post-collection and after being cultured for 1 day (see Fig. 5c).

The cytotoxicity of acetic acid to 2D monolayer culture of MCF7 cells with 1, 2, 5, 10, 20 min exposure was determined by MTT assay (see Fig. 5d).

The relative transcript levels of hormone receptor target genes (ESR1, PGR, GREB1, AR, TMPRSS2), proliferation (Ki67) and apoptosis markers (BCL2) after 2, 5, 10 min exposure to acetic acid and cell culture for 24 h were determined by qPCR (see Fig. 5e).

### Example 6: One-step cell lysis, mRNA capture and reverse transcription (RT), combined with dissociation of alginate after RT process, resulted in cDNA with high yield and desired size.

The inventors tested different strategy for the steps of cell lysis, mRNA capture and RT in alginate:
**a)** Dissociation of alginate before one-step cell lysis, mRNA capture and reverse transcription resulted in no yield of cDNA (see Fig 6a). Dissociation of alginate before cell lysis is a standard in literatures with conventional way of cell lysis and mRNA extraction with e.g. Trizol and spin column. The one-step cell lysis, mRNA capture and RT reaction was shown to work in single cells but not in complex cell microstructures in biomaterial alginate.
**b)** According to the present invention, no dissociation of alginate before one-step cell lysis, mRNA capture and reverse transcription, combined with dissociation of alginate after RT process, surprisingly resulted in cDNA with high yield and desired size at around 1400 bp (see Fig 6b).
**c)** No dissociation of alginate before one-step cell lysis, mRNA capture and RT, no dissociation of alginate after RT process, resulted in no cDNA and residual of primers at smaller size around 100 bp (see Fig 6c).
**d)** The separation of the one-step process into two steps: 1. cell lysis and mRNA capture when alginate is intact, and 2. taking the supernatant surrounding the alginate beads for the RT reaction, resulted in no cDNA (see Fig 6d).

### Example 7: Automated workflow and RNA-seq results for hormone stimulation on primary normal breast tissue microstructures in alginate in miniatured 96 well plate screening format.

The inventors performed an automated workflow with tissue encapsulation in alginate, homogeneous dispensing of alginate into compound pre-coated 96 well plate and the plate plan with two estrogen receptor agonists, E2 and ethinylestradiol (EE2), with 7 concentrations for each compound and 6 technical replicates for each concentration, followed by barcoding, one-step cell lysis, mRNA capture and reverse transcription when alginate is intact and not dissociated, NGS library preparation for RNA-seq analysis (see Fig. 7a).

The fluorescent micrographs showed sufficient diffusion of DNA barcodes modified with fluorescein into alginate beads in 10 minutes (see Fig. 7b).

The fluorescent micrographs showed complete cell lysis for RFP-expressing MCF7 cells in alginate in 20 min. (see Fig. 7c).

Genes expression was analyzed for normal breast tissue microstructures in alginate before and after E2 or EE treatment at 10 pM, 30 pM, 60 pM, 100 pM, 1 nM, 10 nM, 100 nM for 24 hours (see Fig 7d). Estrogen receptor target genes were successfully induced by both E2 and EE and were highlighted in texts on the right of heat map of Fig. 7d.

Having established the platform for high throughput transcriptomics-based hormone and drug testing, the inventors expanded drug and hormone testing on 4 different patient-derived breast tumors. These 4 patient-derived breast tumors showed distinct responses to 3 hormones, E2, R5020 and DHT, that target estrogen, progesterone and androgen receptor after 24 h treatment in alginate (**Fig. 7e****, f**). For METS 15, E2 significantly increased its canonical pathway activity of estrogen response, as well as other pathways related to proliferation and cell cycle progression, such as mTORC1 signaling and E2F targets (**Fig. 7e**). Similarly, DHT significantly increased its canonical pathway activity of androgen response (**Fig. 7e**). Since activated PR has been reported to interact with Jak2 that activate Stat5^{7,8}, the inventors observed similar result that PR agonist R5020 increased Jak/stat pathway activity (**Fig. 7e**). Heatmaps showed detailed and distinct patient responses to E2, R5020 and DHT based on PROGENy pathway activities, where some response trends in common across different patients were also observed, with EGFR, TNFa, Wnt pathways downregulated by E2 for all patients, VEGF pathway upregulated by R5020 for all patients, EGFR, MAPK, NFKb, p52, TNFa and Wnt downregulated by DHT for all patients (**Fig. 7f**).

For drug screening, 4 drugs targeting different signaling pathways in breast cancer were selected, with fulvestrant and tamoxifen as endocrine therapy, palbociclib (CDK4/6i) and alpelisib (PI3Ki) as targeted therapy, and drug combinations of these two categories as combined therapy. Heatmap showed pathway activities of breast tumor microstructures from 3 different patient-derived xenografts after 24 h treatment with fulvestrant (100 nM), tamoxifen (200 nM), albociclib (100 nM) and alpelisib (100 nM) in alginate *ex vivo* (**Fig 7g**). Patient-derived breast tumor METS15 responded to fulvestrant but not to tamoxifen after 24 h treatment in alginate *ex vivo,* with decreased estrogen pathway activity in response to fulvestrant (**Fig. 7h**). The differential responses are consistent with patient mutation profile with *ESR1* mutations for both *D538G* and *E380Q* (**Exosome sequencing data not shown**), which leads to less binding affinity of ER modulator tamoxifen to the changed conformation of ER protein transcribed from mutated *ESR1,* while the efficacy of ER degrader fulvestrant is not affected. As a CDK4/6 inhibitor, palbociclib inhibits CDK4 and CDK6 kinases and prevents cell cycle progression from G1 to S phase⁹, resulting in decreased pathway activity related to cell cycle progression, such as E2F targets and G2M checkpoint. As expected, patient-derived breast tumor T190 showed significantly decreased G2M checkpoint pathway activity and a trend in decreased E2F targets pathway activity after 24 h treatment with palbociclib (**Fig. 7i**). Similar trends in decreased E2F targets and G2M checkpoint were also observed in patient-derived breast tumor T99 and METS15, with decrease in T99 to a greater extent than METS15 (**Fig. 7i**). Approximately 40% of patients with HR-positive, HER2-negative breast cancer harbor *PIK3CA* gene alteration that results in hyperactivation of the alpha isoform (p110α) of phosphatidylinositol 3-kinase (PI3K) and growth and survival of cancer cells^{10,11,12,13,14}. Alpelisib is a α-specific PI3K inhibitor that selectively inhibits p110α and has shown promising efficacy for treating postmenopausal women with HR+/HER2- advanced breast cancer in combination with endocrine therapy^{4,5}. In patient cohort, METS15 and T99 have *PIK3CA* mutation, while the mutation profile of T190 needs to be confirmed. Patient-derived breast tumor T190 showed significant decrease in PI3K/AKT/mTOR signaling pathway activity when treated with alpelisib for 24 h in alginate (**Fig. 7j**). T99 showed a trend of decrease in PI3K/AKT/mTOR pathway activity, which is consistent with the clinical observation that patients haboring *PI3KCA* mutation respond to alpelisib¹¹ (**Fig. 7j**). Although METS15 also has *PI3KCA* mutation, there was no change in PI3K/AKT/mTOR pathway activity after treated with alpelisib (**Fig. 7j**). When combining endocrine therapy with targeted therapy, potential synergisms were observed in fulvestrant plus alpelisib (fa) and tamoxifen plus palbociclib (tp) for METS15 according to decreased MYC targets V1 pathway activity, while fulvestrant was dominant in decreasing estrogen response pathway activities either alone or in combination with palbociclib or alpelisib (**Fig. 7k**). Potential synergisms were also observed in fulvestrant plus alpelisib (fa) and tamoxifen plus alpelisib (ta) for T99 according to E2F targets, G2M checkpoint, mTORC1 signaling, MYC targets V1 and V2 pathway activities (**Fig. 7k**). For T190, fulvestrant plus palbociclib (fp) showed synergism in estrogen pathway activity, but not in other pathways related to cell cycle progression and proliferation, while fulvestrant plus alpelisib (fa) and tamoxifen plus alpelisib (ta) significantly decreased pathway activities of G2M checkpoint, mTORC1 signaling and MYC targets V1 compared to control, but not reached synergism compared to single drug (**Fig. 7k**). However, T190 showed effective response to single drug tamoxifen, palbociclib and alpelisib with decreased pathway activities in estrogen response, G2M checkpoint, mTORC1 signaling and MYC targets V1 (**Fig. 7k**), which potentially means that single drug would be sufficient to reach therapeutic efficacy for this treatment-naive patient. Taking into consideration the different cell origins, treatment history and mutation status of 3 patients, where METS15 was from metastatic site of a patient treated with chemotherapy, endocrine and targeted therapies for 9 years, and T99 and T190 were from primary site of treatment-naive patients, these data suggested distinct patient response profiles to the same drug, highlighting the importance of drug testing for personalized medicine.

Taken together, this platform provides a versatile approach for high throughput hormone and drug testing with transcriptomic readout, and holds potential for deciphering the underlying mechanism of different patient responses to enable personalized medicine for optimal therapeutic outcome.

### REFERENCES

**1.** Tanos, Tamara, et al. Science translational medicine 5.182 (2013): 182ra55-182ra55. « Tissue microstructure culture ».
**2.** Cartaxo, Ana Luisa, et al. Journal of Experimental & Clinical Cancer Research 39.1 (2020): 1-14 "Alginate -based breast cancer tissue culture".
**3.** Ethan S. Sokol et al, Breast Cancer Research 18.1 (2016): 1-13. "Hydrogel-based breast cancer tissue culture"
**4.** Reichard A, Asosingh K. Best Practices for Preparing a Single Cell Suspension from Solid Tissues for Flow Cytometry. Cytometry A. 2019 Feb;95(2):219-226. doi: 10.1002/cyto.a.23690. Epub 2018 Dec 6. PMID: 30523671; PMCID: PMC6375754.
**5.** The Worthington Tissue Dissociation Guide https://www.worthington-biochem.com/tissuedissociation/default.html (visited on 14/02/22)
**6.** Estrada MF, Rebelo SP, Davies EJ, Pinto MT, Pereira H, Santo VE, Smalley MJ, Barry ST, Gualda EJ, Alves PM, Anderson E, Brito C. Modelling the tumour microenvironment in long-term microencapsulated 3D co-cultures recapitulates phenotypic features of disease progression. Biomaterials. 2016 Feb;78:50-61. doi: 10.1016/j.biomaterials.2015.11.030. Epub 2015 Nov 19. PMID: 26650685.
**7.** Wright, R. H. G., Vastolo, V., Oliete, J. Q., Carbonell-Caballero, J. & Beato, M. Signalling Network of Breast Cancer Cells in Response to Progesterone. bioRxiv 2020.11.03.366401 (2020) doi:10.1101/2020.11.03.366401.
**8.** Hagan, C. R., Knutson, T. P. & Lange, C. A. A Common Docking Domain in Progesterone Receptor-B links DUSP6 and CK2 signaling to proliferative transcriptional programs in breast cancer cells. Nucleic Acids Res. 41, 8926-8942 (2013).
**9.** Turner, N. C. et al. Palbociclib in Hormone-Receptor-Positive Advanced Breast Cancer. N. Engl. J. Med. 373, 209-219 (2015).
**10.** Chang, D. Y., Ma, W. L. & Lu, Y. S. Role of Alpelisib in the Treatment of PIK3CA-Mutated Breast Cancer: Patient Selection and Clinical Perspectives. Ther. Clin. Risk Manag. 17, 193 (2021).
**11.** André, F. et al. Alpelisib for PIK3CA -Mutated, Hormone Receptor-Positive Advanced Breast Cancer . N. Engl. J. Med. 380, 1929-1940 (2019).
**12.** Koboldt, D. C. et al. Comprehensive molecular portraits of human breast tumours. Nat. 2012 4907418 490, 61-70 (2012).
**13.** Mollon, L. et al. Abstract 1207: A systematic literature review of the prevalence of PIK3CA mutations and mutation hotspots in HR+/HER2- metastatic breast cancer. Cancer Res. 78, 1207-1207 (2018).
**14.** Goncalves, M. D., Hopkins, B. D. & Cantley, L. C. Phosphatidylinositol 3-Kinase, Growth Disorders, and Cancer. N. Engl. J. Med. 379, 2052-2062 (2018).

## Claims

1. A method for analyzing cellular responsiveness to hormones or drugs in a tissue sample from a patient or in a patient-derived tissue sample, comprising the steps of:
a) Mechanical and/or enzymatic digestion of said tissue sample to produce tissue microstructures;
b) Encapsulation of said tissue microstructures in alginate;
c) Exposing the encapsulated tissue microstructure to hormones or drugs;
d) Cell lysis, mRNA capture and reverse transcription in one-step;
e) Alginate dissociation;
f) RNA sequencing;
wherein step d) is performed with the alginate still crosslinked and intact.

2. The method according to claim 1, wherein in step a) the proteins from the extracellular matrix and cells from stroma of the tissue sample are preserved as a natural surround environment for the microstructures.

3. The method according to claim 1 or 2, wherein in step b) no supplementary hormones or growth factors are added during the encapsulation.

4. The method according to any of the claim 1 to 3, wherein in step b) no collagen-based biomaterial is added during the encapsulation.

5. The method according to any of the claim 1 to 4, wherein in step b) the tissue microstructures are encapsulated in alginate beads having a diameter of between 10 to 1200 µm, preferably between 40 to 800 µm, more preferably between 50 to 500 µm.

6. The method according to any of the claim 1 to 5, wherein the encapsulated tissue microstructures are maintained in culture for 3h to 23 days, preferably 5h to 14 days, more preferably 12h to 10 days, even more preferably 24h to 7 days prior to step c).

7. The method according to any of the claim 1 to 6, wherein in step b) encapsulated tissue microstructures are in static culture or cultured without agitation.

8. The method according to any of the claim 1 to 7, wherein said tissue sample is selected from the group consisting of epithelium tissue, connective tissue, cartilage tissue, bone tissue, muscle tissue, nerve tissue, blood vessel tissue, heart tissue, lymphatic system tissue, respiratory tract tissue, oesophagus tissue, urinary tract tissue, endocrine system tissue, reproductive system tissue, breast tissue and cancer tissue, preferably breast tissue or breast cancer tissue sample.

9. The method according to any of the claim 1 to 8, wherein said tissue sample is an estrogen receptor positive (ER+) breast cancer tissue sample.

10. The method according to any of the claim 1 to 9, wherein said hormones are selected from the group consisting of receptor agonists or receptor antagonists, preferably estrogen receptor agonist or antagonist, progesterone receptor agonist or antagonist, androgen receptor agonist or antagonist and glucocorticoid receptor agonist or antagonist; and said drugs are selected from the group consisting of FDA-approved drugs and new drugs under clinical trials.

11. The method according to any of the claim 1 to 10, wherein said hormones are selected from the group consisting of E2, EE2, Progesterone, R5020, DHT, dexamethasone, and progestins such as levonorgestrel, chlormadinone acetate, desogestrel, cyproterone acetate, gestodene and drospirenone,

12. The method according to any of the claim 1 to 11, wherein said drugs are selected from the group consisting of are tamoxifen, fulvestrant, mifepristone, enzalutamide, palbociclib, abemaciclib, ribociclib, alpelisib, everolimus, olaparib, capivasertib, ER protac, AR protac and PR protac.

13. The method according to any of the claim 1 to 12, further comprising step g) of performing one or more further assays and/or one or more further analytical techniques to determine the tissue responsiveness to hormones or drugs, preferably said further one or more assays or analytical techniques assess one or more of cell viability, cell proliferation, cell apoptosis, gene expression changes in the said tissue in response to hormones and drugs.

14. The method according to any of the claim 1 to 13, wherein the one or more assays or analytical techniques are selected from MTT assay, Live/Dead staining, bioluminescence assay, immunofluorescent staining (IF), immunohistochemistry staining (IHC), western blot, polymerase chain reaction (PCR), RNA-seq and/or the one or more assays or analytical techniques classify the said tissue sample as responsive or non-responsive to the hormones and/or drugs.

15. The method according to any of the claim 1 to 14, for use in hormone and drug testing for personalized medicine, drug discovery, drug development, pre-clinical or clinical studies.

## Patentansprüche

1. Verfahren zur Analyse der zellulären Reaktion auf Hormone oder Arzneimittel in einer Gewebeprobe von einem Patienten oder in einer Patienten-abgeleiteten Gewebeprobe, das die Schritte umfasst:
a) mechanische und/oder enzymatische Verdauung der Gewebeprobe zur Herstellung von Gewebemikrostrukturen;
b) Verkapselung der Gewebemikrostrukturen in Alginat;
c) Exposition der verkapselten Gewebemikrostrukturen gegenüber Hormonen oder Arzneimitteln;
d) Zelllyse, mRNA-Anreicherung und reverse Transkription in einem Schritt;
e) Dissoziation des Alginats;
f) RNA-Sequenzierung;
wobei Schritt d) durchgeführt wird, während das Alginat noch vernetzt und intakt ist.

2. Verfahren nach Anspruch 1, wobei in Schritt a) die Proteine aus der extrazellulären Matrix und Zellen aus dem Stroma der Gewebeprobe als natürliche Umgebung für die Mikrostrukturen erhalten bleiben.

3. Verfahren nach Anspruch 1 oder 2, wobei in Schritt b) während der Verkapselung keine zusätzlichen Hormone oder Wachstumsfaktoren hinzugefügt werden.

4. Verfahren nach einem der Ansprüche 1 bis 3, wobei in Schritt b) während der Verkapselung kein kollagenbasiertes Biomaterial hinzugefügt wird.

5. Verfahren nach einem der Ansprüche 1 bis 4, wobei in Schritt b) die Gewebemikrostrukturen in Alginatkügelchen mit einem Durchmesser von 10 bis 1200 µm, vorzugsweise 40 bis 800 µm, besonders bevorzugt 50 bis 500 µm, verkapselt werden.

6. Verfahren nach einem der Ansprüche 1 bis 5, wobei die verkapselten Gewebemikrostrukturen vor Schritt c) 3 Stunden bis 23 Tage, vorzugsweise 5 Stunden bis 14 Tage, besonders bevorzugt 12 Stunden bis 10 Tage, noch bevorzugter 24 Stunden bis 7 Tage in Kultur gehalten werden.

7. Verfahren nach einem der Ansprüche 1 bis 6, wobei die verkapselten Gewebemikrostrukturen in Schritt b) in statischer Kultur oder ohne Agitation kultiviert werden.

8. Verfahren nach einem der Ansprüche 1 bis 7, wobei die Gewebeprobe aus der Gruppe bestehend aus Epithelgewebe, Bindegewebe, Knorpelgewebe, Knochengewebe, Muskelgewebe, Nervengewebe, Blutgefäßgewebe, Herzgewebe, Gewebe des lymphatischen Systems, Atemwegsgewebe, Speiseröhrengewebe, Harnwegsgewebe, Gewebe des endokrinen Systems, Gewebe des Fortpflanzungssystems, Brustgewebe und Krebsgewebe, vorzugsweise Brustgewebe- oder Brustkrebsgewebeprobe ausgewählt ist.

9. Verfahren nach einem der Ansprüche 1 bis 8, wobei es sich bei der Gewebeprobe um eine Östrogenrezeptor-positive (ER+) Brustkrebsgewebeprobe handelt.

10. Verfahren nach einem der Ansprüche 1 bis 9, wobei die Hormone aus der Gruppe bestehend aus Rezeptoragonisten oder Rezeptorantagonisten, vorzugsweise Östrogenrezeptoragonist oder -antagonist, Progesteronrezeptoragonist oder -antagonist, Androgenrezeptoragonist oder -antagonist und Glukokortikoidrezeptoragonist oder - antagonist ausgewählt sind; und die Arzneimittel aus der Gruppe bestehend aus FDAzugelassenen Arzneimitteln und neuen Arzneimitteln in klinischen Studien ausgewählt sind.

11. Verfahren nach einem der Ansprüche 1 bis 10, wobei die Hormone aus der Gruppe bestehend aus E2, EE2, Progesteron, R5020, DHT, Dexamethason und Gestagenen wie Levonorgestrel, Chlormadinonacetat, Desogestrel, Cyproteronacetat, Gestoden und Drospirenon ausgewählt sind.

12. Verfahren nach einem der Ansprüche 1 bis 11, wobei die Arzneimittel aus der Gruppe bestehend aus Tamoxifen, Fulvestrant, Mifepriston, Enzalutamid, Palbociclib, Abemaciclib, Ribociclib, Alpelisib, Everolimus, Olaparib, Capivasertib, ER Protac, AR Protac und PR Protac ausgewählt sind.

13. Verfahren nach einem der Ansprüche 1 bis 12, das ferner Schritt g) der Durchführung eines oder mehrerer weiterer Assays und/oder einer oder mehrerer weiterer analytischer Techniken zur Bestimmung der Gewebereaktion auf Hormone oder Arzneimittel umfasst, wobei die weiteren ein(e) oder mehrere Assays oder analytischer Techniken vorzugsweise eine oder mehrere der Zellviabilität, Zellproliferation, Zellapoptose oder Genexpressionsänderungen im Gewebe als Reaktion auf Hormone und Arzneimittel bewerten.

14. Verfahren nach einem der Ansprüche 1 bis 13, wobei die einen oder mehrere Assays oder analytischen Techniken aus MTT-Assay, Lebend-/Totfärbung, Biolumineszenz-Assay, Immunfluoreszenzfärbung (IF), Immunhistochemie-Färbung (IHC), Western Blot, Polymerase-Kettenreaktion (PCR), RNA-Seq ausgewählt sind, und/oder die einen oder mehrere Assays oder analytischen Techniken die Gewebeprobe als responsiv oder nichtresponsiv gegenüber den Hormonen und/oder Arzneimitteln klassifizieren.

15. Verfahren nach einem der Ansprüche 1 bis 14, zur Verwendung in der Hormon- und Arzneimitteltestung für personalisierte Medizin, Arzneimittelforschung, Arzneimittelentwicklung, präklinische oder klinische Studien.

## Revendications

1. Procédé d'analyse de réactivité cellulaire à des hormones ou des médicaments dans un échantillon tissulaire provenant d'un patient ou dans un échantillon tissulaire dérivé d'un patient, comprenant les étapes suivantes :
a) la digestion mécanique et/ou enzymatique dudit échantillon tissulaire pour produire des micro-structures tissulaires ;
b) l'encapsulation desdites micro-structures tissulaires dans de l'alginate ;
c) l'exposition de la micro-structure tissulaire encapsulée à des hormones ou des médicaments ;
d) la lyse cellulaire, la capture d'ARNm et la transcription inverse en une seule étape ;
e) la dissociation d'alginate ;
f) le séquençage d'ARN ;
dans lequel l'étape d) est réalisée avec de l'alginate encore réticulé et intact.

2. Procédé selon la revendication 1, dans lequel, à l'étape a), les protéines provenant de la matrice extracellulaire et les cellules provenant du stroma de l'échantillon tissulaire sont préservées en tant qu'environnement ambiant naturel pour les micro-structures.

3. Procédé selon la revendication 1 ou 2, dans lequel, à l'étape b), aucune hormone supplémentaire ni aucun facteur de croissance supplémentaire n'est ajouté(e) pendant l'encapsulation.

4. Procédé selon l'une quelconque des revendications 1 à 3, dans lequel, à l'étape b), aucun biomatériel à base de collagène n'est ajouté pendant l'encapsulation.

5. Procédé selon l'une quelconque des revendications 1 à 4, dans lequel, à l'étape b), les micro-structures tissulaires sont encapsulées dans des billes d'alginate ayant un diamètre compris entre 10 et 1 200 µm, de préférence entre 40 et 800 µm, plus préférentiellement entre 50 et 500 µm.

6. Procédé selon l'une quelconque des revendications 1 à 5, dans lequel les micro-structures tissulaires encapsulées sont maintenues en culture pendant 3 h à 23 jours, de préférence 5 h à 14 jours, plus préférentiellement 12h à 10 jours, encore plus préférentiellement 24 h à 7 jours avant l'étape c).

7. Procédé selon l'une quelconque des revendications 1 à 6, dans lequel, à l'étape b), des micro-structures tissulaires encapsulées sont en culture statique ou mises en culture sans agitation.

8. Procédé selon l'une quelconque des revendications 1 à 7, dans lequel ledit échantillon tissulaire est choisi parmi le groupe consistant en un tissu épithélial, tissu conjonctif, tissu cartilagineux, tissu osseux, tissu musculaire, tissu nerveux, tissu vasculaire, tissu cardiaque, tissu du système lymphatique, tissu des voies respiratoires, tissu de l'œsophage, tissu des voies urinaires, tissu du système endocrinien, tissu du système reproducteur, tissu mammaire et tissu cancéreux, de préférence un échantillon de tissu mammaire ou de tissu de cancer du sein.

9. Procédé selon l'une quelconque des revendications 1 à 8, dans lequel ledit échantillon tissulaire est un échantillon de tissu de cancer du sein à récepteurs d'œstrogènes positifs (ER+).

10. Procédé selon l'une quelconque des revendications 1 à 9, dans lequel lesdites hormones sont choisies parmi le groupe consistant en des agonistes de récepteurs ou antagonistes de récepteurs, de préférence agoniste ou antagoniste de récepteurs d'œstrogènes, agoniste ou antagoniste de récepteurs de progestérone, agoniste ou antagoniste de récepteurs d'androgènes et agoniste ou antagoniste de récepteurs de glucocorticoïdes ; et lesdits médicaments sont choisis parmi le groupe consistant en des médicaments approuvés par la FDA et de nouveaux médicaments en cours d'essais cliniques.

11. Procédé selon l'une quelconque des revendications 1 à 10, dans lequel lesdites hormones sont sélectionnées parmi le groupe consistant en E2, EE2, progestérone, R5020, DHT, dexaméthasone et progestatifs, par exemple lévonorgestrel, acétate de chlormadinone, désogestrel, acétate de cyprotérone, gestodène et drospirénone.

12. Procédé selon l'une quelconque des revendications 1 à 11, dans lequel lesdits médicaments sont choisis parmi le groupe consistant en tamoxifène, fulvestrant, mifépristone, enzalutamide, palbociclib, abémaciclib, ribociclib, alpelisib, évérolimus, olaparib, capivasertib, protac ER, protac AR et protac PR.

13. Procédé selon l'une quelconque des revendications 1 à 12, comprenant en outre l'étape g) de réalisation d'un ou plusieurs autres essais et/ou d'une ou plusieurs autres techniques analytiques pour déterminer la réactivité tissulaire à des hormones ou des médicaments, de préférence lesdits un ou plusieurs autres essais ou techniques analytiques évaluent un ou plusieurs parmi une viabilité cellulaire, une prolifération cellulaire, une apoptose cellulaire, des changements d'expression génique dans ledit tissu en réponse à des hormones et des médicaments.

14. Procédé selon l'une quelconque des revendications 1 à 13, dans lequel les un ou plusieurs essais ou techniques analytiques sont choisis parmi un essai MTT, une coloration vivant/mort, un essai par bioluminescence, une coloration par immunofluorescence (IF), une coloration immunohistochimique (IHC), un transfert Western, une réaction en chaîne par polymérase (PCR), un séquençage d'ARN et/ou les un ou plusieurs essais ou techniques analytiques classifient ledit échantillon tissulaire comme étant réactif ou non réactif aux hormones et/ou aux médicaments.

15. Procédé selon l'une quelconque des revendications 1 à 14, pour une utilisation dans des tests hormonaux et médicamenteux pour une médecine personnalisée, une découverte de médicaments, un développement de médicaments, des études pré-cliniques ou cliniques.
